# EUROPEAN PATENT SPECIFICATION

(11) **EP 2 163 653 B1**
(45) Date of publication and mention of the grant of the patent: **27.03.2013**
(21) Application number: 09015418.8
(22) Date of filing: 10.11.2004
(51) Int. Cl.: C12Q 1/70, G01N 33/00, C07H 21/02, C12Q 1/68, C08F 2/48, G03F 7/028

(54) **Nucleic acid detection method having increased sensitivity**
Nukleinsäurenachweisverfahren mit erhörter Empfindlichkeit
Procédé de détection d'acides nucléiques avec sensibilité accrue

(30) Priority: 10.11.2003 US 518816 P
(43) Date of publication of application: 17.03.2010
(62) Divisional of application: 04818676.1
(73) Proprietor: Geneohm Sciences, Inc., San Diego, CA 92121 (US)
(72) Inventor: Crothers, Donald M., Northford Connecticut 06472 (US); Holmlin, Erik R., Rancho Santa Fe CA 92067 (US); Zhang, Honghua, San Diego California 92130 (US); Shi, Chunnian, San Diego California 92130 (US)
(74) Representative: Raynor, Simon Mark

(56) References cited:
- WO-A-03/064692
- WO-A2-03/066817
- COLLINS M L ET AL: "A branched DNA signal amplification assay for quantification of nucleic acid targets below 100 molecules/ml" NUCLEIC ACIDS RESEARCH, OXFORD UNIVERSITY PRESS, SURREY, GB, vol. 25, no. 15, 1997, pages 2979-2984, XP002208495 ISSN: 0305-1048
- CHEN J ET AL: "DETECTION OF VEROTOXIGENIC ESCHERICHIA COLI BY MAGNETIC CAPTURE-HYBRIDIZATION PCR" APPLIED AND ENVIRONMENTAL MICROBIOLOGY, AMERICAN SOCIETY FOR MICROBIOLOGY, US, vol. 64, no. 1, 1 January 1998 (1998-01-01), pages 147-152, XP000908818 ISSN: 0099-2240
- NALLUR G ET AL: "Signal amplification by rolling circle amplification on DNA microarrays" NUCLEIC ACIDS RESEARCH, OXFORD UNIVERSITY PRESS, SURREY, GB, vol. 29, no. 23, 1 December 2001 (2001-12-01), page E118, XP002387058 ISSN: 0305-1048
- LIZARDI P M ET AL: "MUTATION DETECTION AND SINGLE-MOLECULE COUNTING USING ISOTHERMAL ROLLING-CIRCLE AMPLIFICATION" NATURE GENETICS, NEW YORK, NY, US, vol. 19, no. 3, July 1998 (1998-07), pages 225-232, XP000856939 ISSN: 1061-4036
- CALIN ANDRAS S ET AL: "STRATEGIES FOR SIGNAL AMPLIFICATION IN NUCLEIC ACID DETECTION" MOLECULAR BIOTECHNOLOGY, TOTOWA, NJ, US, vol. 19, no. 1, 2001, pages 29-44, XP001031378 ISSN: 1073-6085

## Description

### Background of the Invention

### Field of the Invention

The present invention relates to nucleic acid detection methods having increased sensitivity. In some advantageous embodiments, the method includes electrochemical detection of a catalytic cycle between a synthetically elongated nucleic acid and an electrode surface.

### Description of the Related Art

Hybridization of polynucleotides to other polynucleotides having at least a portion of complementary nucleotide sequence by Watson-Crick base pairing is a fundamental process useful in a wide variety of research, medical, and industrial applications. Detecting the hybridization of a probe to a polynucleotide containing a target sequence is useful for gene expression analysis, DNA sequencing, and genomic analysis. Particular uses include identification of disease-related polynucleotides in diagnostic assays, screening for novel target polynucleotides in a sample, identification of specific target polynucleotides in mixtures of polynucleotides, identification of variant sequences, genotyping, amplification of specific target polynucleotides, and therapeutic blocking of inappropriately expressed genes, *e*.*g*. as described in Sambrook et al., Molecular Cloning: A Laboratory Manual, 2nd Edition (Cold Spring Harbor Laboratory, New York, 1989); Keller and Manak, DNA Probes, 2nd Edition (Stockton Press, New York, 1993); Milligan et al., 1993, J Med Chem, 36: 1923-1937; Drmanac et al., 1993. Science, 260: 1649-1652; Bains, 1993, J DNA Seq Map, 4: 143-150.

Immobilized probes are useful for detecting polynucleotides containing a target nucleotide sequence, where each immobilized probe is functionally connected to a support and the hybridization of a polynucleotide to the immobilized probe can be detected. Most commonly, DNA probes are used to detect polynucleotides containing a target nucleotide sequence complementary to the probe sequence. The support for immobilized probes may be a flat surface, often called a "chip," or the support may be the surface of a bead or other particle. Probes are usually immobilized in a known arrangement, or array, which provides a medium for matching known and unknown polynucleotides based on base-pairing rules. Preferably, the process of identifying the unknowns identified using a probe array is automated. Microarrays having a large number of immobilized probes of known identity are used to determine complementary binding, allowing massively parallel studies of gene expression and gene discovery. For example, an experiment with a single DNA chip can provide researchers information on thousands of genes simultaneously. For example, Hashimoto *et al*. disclose an array of immobilized single-stranded probes wherein at least one probe has a nucleotide sequence complementary to the target gene(s) to be detected, such that each probe is immobilized onto the surface of an electrode or the tip of an optical fiber and an electrochemically or optically active substance capable of binding to doublestranded nucleic acid is used to detect hybridization of target genes to complementary immobilized probes (U.S. Pat. Nos. 5,776,672 and 5,972,692).

Another method to detect whether nucleic acid hybridization has occurred is to detect a signal reflecting the quantity of counterions surrounding the nucleic acid. Accordingly, hybridized nucleic acid would tend to be surrounded by more counterions than would single stranded nucleic acid. The counterions are typically detected by an electrochemical reaction, for example by reduction of a trivalent ion to divalent; in this way, the counterions function as an electron transfer species.

Electrochemical quantitation is described in A.B. Steel et al., Electrochemical Quantitation of DNA Immobilized on Gold, Anal. Chem. 70:4670-77 (1998). In this publication, Steel *et al*. describe the use of cobalt (III) trisbipyridyl and ruthenium (III) hexaamine as species which interact with surface-immobilized DNA.

### Universal chips

Under some circumstances, a drawback to chip technology is that each chip must be manufactured specifically for the sequences to be detected, with a set of immobilized probes that are designed to be complementary to specific sequences to be detected. Chips specific for a single organism require a large manufacturing investment, and the chips can only be used for a narrowly defined range of samples. In contrast, a "universal chip" or "universal array" is organism-independent because the probes are not targeted to organism-specific sequences or products. Chips specific for a specific tissue, physiological condition, or developmental stage, often used for gene expression analysis, can likewise require a substantial manufacturing investment for use with a limited range of samples. A universal chip provides an unrestricted approach to studying tissues, physiological conditions, or developmental stages of interest. Manufacturing quality control can be improved by using a universal chip for polynucleotide detection.

One approach to universal chip design involves attaching a set of oligonucleotide probes to a chip surface, where the set of oligonucleotide probes includes all possible sequences of oligonucleotides that are 5, 6, 7, 8, 9, 10 or more nucleotides in length. The probes needed for these arrays can be designed using a simple combinatorial algorithm. The chip is incubated with a mixture that may contain DNA, cDNA, RNA or other hybridizable material, and hybridization to each probe of known sequence is measured. However, the specificity of such an array may be impaired because different sequences may have different requirements for stringent hybridization. In addition, such a universal array does not prevent false positives resulting from frameshifting where, for example in a universal array having probes that are six nucleotides long, the final four nucleotides of a sample polynucleotide may hybridize to the complementary final four nucleotides of a six-nucleotide probe, but the same sample polynucleotide would not hybridize to the entire six-nucleotide probe sequence.

Suyama et al. (2000, Curr Comp Mol Biol 7:12-13) disclose a universal chip system for gene expression profiling of a sample, where the chip system utilizes "DNA computing" instead of binding of transcripts to probes. The DNA computing system of Suyama *et al*. indirectly determines which transcripts are present by measuring binding of coded adapters to a universal set of immobilized probes on the universal chip. Only those coded adapters with a region complementary to a region of a transcript present in a sample will undergo the subsequent manipulations and the processing steps that generate adapters capable of binding to probes on the universal chip.

### Tags

An alternative approach to manufacturing a universal chip involves using a set of tag sequences that do not naturally occur in the target polynucleotides, where the tags bind to complementary probes on a universal chip. Tags for such uses are sometimes known as "address tags" or "zip codes" or are considered to be analogous to "bar codes" for identifying targets. Detection, identification, tracking, sorting, retrieving or other manipulations are then directed at tag sequences and not the sequences of the target polynucleotides. Oligonucleotide tags may be covalently attached to or incorporated into polynucleotides. Tags may become associated with a polynucleotide by hybridization of a separate oligonucleotide which functions as a linker by virtue of having at least two domains, one with a tag sequence complementary to a probe and one with sequence complementary to at least a portion of the target polynucleotide. Systems employing oligonucleotide tags have been proposed as means for manipulating and identifying individual molecules in complex mixtures, for example to detect polynucleotides having target nucleotide sequences, or as an aid to screening genomic, cDNA, or combinatorial libraries for drug candidates. Brenner and Lerner, 1992, Proc Natl Acad Sci, 89: 5381-5383; Alper, 1994, Science, 264: 1399-1401; Needels et al., 1993, Proc Nat Acad Sci, 90: 10700-10704.

### Spurious signals

The usefulness of tagged polynucleotides depends in large part on success in achieving specific hybridization between a tag and its complementary probe immobilized to a surface. For an oligonucleotide tag to successfully identify a polynucleotide, the number of false positive and false negative signals should be minimized. Unfortunately, spurious signals are not uncommon because base pairing and base stacking free energies can vary widely among nucleotide sequences in a duplex or triplex structure. For example, a tag-probe duplex having a different number of guanosine-cytosine (G-C) pairs than another duplex will have a different melting temperature, such that tag-probe duplexes with differing G-C ratios will have different stringency requirements for hybridization. In addition, a tag-probe duplex consisting of a repeated sequence of adenosine (A) and thymidine (T) bound to its complement may have less stability than a duplex of equal length consisting of a repeated sequence of G and C bound to a partially complementary target containing a mismatch, due to differences in stacking energy. Special reagents are often required to balance these differences in stacking energy.

Spurious signals can also result from "frameshifting" as described above. This problem has been addressed by employing a "comma-less" code, which ensures that a probe out of register (frameshifted) with respect to its complementary tag would result in a duplex with one or more mismatches for each of its codons, which forms an unstable duplex.

In view of the above problems with spurious signals, researchers have developed various oligonucleotide-based tagging systems which provide a sufficient repertoire of tags, but which also minimize the occurrence of false positive and false negative signals without the need to employ special reagents for altering natural base pairing and base stacking free energy differences, or elaborate encoding systems for comma-less codes. Such tagging systems find applications in many areas, including construction and use of combinatorial chemical libraries, large-scale mapping and sequencing of DNA, genetic identification, and medical diagnostics.

Brenner *et al*. disclose a "universal" chip system that attaches tags to the ends of polynucleotide fragments through reactive moieties, where spurious signals are avoided by designing a repertoire of multi-subunit oligonucleotide tags with sequences such that the stability of any mismatched duplex or triplex between a tag and a complement to another tag is far lower than that of any perfectly matched duplex between the tag and its own complement. U.S. Pat. Nos. 5,604,097, 5,654,413, 5,846,719, 5,863,722, 6,140,489, 6,150,516, 6,172,214, 6,172,218, 6,352,828, 6,235,475. Morris et al. (U.S. Pat. No. 6,458,530, EP 0799897) disclose the use of tags and arrays of complementary probes to label and track compositions including cells and viruses, and to facilitate analysis of cell and viral phenotypes.

An alternate approach involves multicomponent tagging systems where tags are not attached to polynucleotides but rather, are found on separate components that are hybridized to the polynucleotides in order to adapt, index, and/or detect polynucleotides having a defined nucleotide sequence. The method disclosed in U.S. Pat. No. 6,261,782 and related patents and applications (Lizardi *et al*.) permits the user to sort and identify target polynucleotides in a sample by generating "sticky ends" using nucleic acid cleaving reagents, indexing the cleaved polynucleotide fragments into sets by adding adapter-indexer oligonucleotides with ends complementary to various sticky ends to the sample, adding ligator-detector oligonucleotides with sticky ends complementary to the sticky ends of adapter-indexers, hybridizing the entire sample with a plurality of detector probes, covalently coupling the ligator-detectors to the detector probes, and finally detecting coupling of ligator-detectors to the detector probes.

Another multicomponent system is disclosed by Balch et al., in U.S. Pat. No. 6,331,441, using a bifunctional linker with a domain that hybridizes to an immobilized capture probe in a universal array and a domain that hybridizes to an analyte containing a target. Balch *et al*. also discloses amplification of a target polynucleotide to generate amplicons containing both target sequence and a unique universal sequence complementary to a capture probe, where the unique universal sequence may be introduced through PCR or LCR primers (U.S. Pat. No. 6,331,441).

In some circumstances, it is desirable to obtain a sensitivity of nucleic acid detection which is greater than that afforded by currently existing amperometry techniques. For example, using existing technology it is sometimes difficult to distinguish whether target strands have hybridized to probes, particularly in cases where a very small amount of target exists in a sample or where it is impossible or impractical to amplify the target (as by PCR or RCA) prior to hybridization. Accordingly, there is an unmet need in the art for nucleic acid detection methods having increased sensitivity.

WO 03/064692 A discloses the detection and quantification of different nucleic acid sequences within a sample by hybridizing a target nucleic acid to a capture probe, hybridizing a counting probe to the target nucleic acid, ligating the capture probe to the counting probe, hybridizing a single-strand circular DNA to the counting probe, and then amplifying the circular DNA to allow detection of the target nucleic acid.

WO 03/066817 A2 discloses methods of amplifying and detecting DNA by applying variations of rolling circle amplification to several detection platforms.

### Summary of the Invention

An aspect of the present invention is a method for determining whether a sample contains a target polynucleotide, including the steps of: placing a sample in contact with a detection zone containing a capture probe polynucleotide complementary to at least a portion of a sequence in the target polynucleotide under conditions which permit the target polynucleotide to hybridize to the capture probe polynucleotide, wherein said detection zone comprises an electrode; extending said target polynucleotide; hybridizing a first bridge nucleic acid to the extended target polynucleotide hybridized to the capture probe polynucleotide, wherein said first bridge nucleic acid comprises a nucleotide sequence complementary to a nucleotide sequence in said target polynucleotide and a nucleotide sequence complementary to a first circular nucleic acid; extending said first bridge nucleic acid such that said extended first bridge nucleic acid is non-contiguous with said probe polynucleotide; and determining whether a signal indicative of the presence of the target polynucleotide in the sample has been generated, wherein the signal is generated by a catalytic detection reagent which can produce a plurality of signals without being exhausted.

### Brief Description of the Drawings

**FIG. 1** shows catalytic cycling using HRP and TMB in the conversion of H₂O₂, to water.
**FIG. 2A** shows a detection moiety electrostatically associated with hybridized nucleic acid.
**FIG. 2B** shows a detection moiety associated with hybridized nucleic acid using a detector probe.
**FIG. 3A** illustrates on-chip amplification using head-to-tail polymerization.
**FIG. 3B** illustrates on-chip amplification using rolling circle amplification.
**FIG. 3C** illustrates on-chip amplification using a branch technique in conjunction with rolling circle amplification.
**FIG. 3D** illustrates on-chip amplification using a hyperbranch technique in conjunction with rolling circle amplification.
**FIG. 4** illustrates signal amplification for direct pathogen detection using both on-chip amplification and catalytic detection.
**FIG. 5** shows a process that uses both on-chip amplification and hybridization of a detector probe to which HRP can couple.
**FIG. 6** shows an example of direct detection of hybridization using in-situ ligation and RCA.
**FIG. 7** shows an example of pathogen detection.
**FIG. 8** shows a comparison of catalytic detection using HRP with standard electrochemical detection using a ruthenium complex.
**FIG. 9** shows the relationship between the concentration of a nucleic acid target in a sample and detectable current.
**FIG. 10** shows nucleic acid sequences used as targets, capture probes, and bridges in an HPV detection assay.
**FIG. 11** shows the results of an assay wherein the concentrations of the target, bridge, and circle nucleic acid strands were varied.
**FIG. 12** shows an RCA procedure in which a circle is captured by a capture probe and multistage RCA is conducted to amplify the captured nucleic acid.
**FIG. 13** shows the results of an assay using two-stage RCA with detection using HRP.
**FIG. 14** shows the results of an assay using two-stage RCA with detection using ruthenium hexamine.

### Detailed Description of the Preferred Embodiments

The present invention is generally related to methods of detecting the presence of a target nucleic acid having a particular sequence in a sample. Some preferred embodiments of the present invention include the detection of the target nucleic acid using polynucleotide hybridization in a detection zone. In some advantageous embodiments, such a detection zone includes an electrode surface and is located on an assay chip, preferably a universal assay chip.

In some embodiments, the target nucleic acid is detected by measuring an electric signal (such as a current or a voltage) resulting from the hybridization of the target nucleic acid to a probe nucleic acid on the assay chip. In particular embodiments, the electric current may be generated by associating a detection reagent, such as an agent capable of being oxidized or reduced, with the target nucleic acid which is hybridized to the probe nucleic acid. In some embodiments, the the detection reagent participates in the transfer of electrons only one time; in some embodiments, however, the detection reagent can participate in redox reactions repeatedly without being exhausted. If a detection reagent can participate repeatedly, it can be referred to as a "catalytic" detection reagent. In some embodiments, a catalytic detection reagent is a redox moiety, such an enzyme. In some additional embodiments, the amplitude of the electric current resulting from hybridization of the target nucleic acid to the probe is increased by associating many detection agents with each hybridized target nucleic acid and/or utilizing detection reagents which participate in catalytic oxidation/reduction reactions.

In some embodiments, the method is based on detecting the presence of a negatively charged target strand through interaction of the target with a detection reagent, such as a redox moiety, linked to polyamine, other cationic peptides, or cationic polymers which associate with the target nucleic acid via electrostatic interactions. In other embodiments, the method is based on the detection of a target strand based on its sequence-specific hybridization to an additional probe, a "detector probe," that is in turn affiliated with a redox moiety. Detector probes are sometimes referred to as "signal probes." Examples of redox moieties affiliated with detector probes are discussed herein.

Various techniques and electron transfer species useful for nucleic acid detection are disclosed in WO 2004/044549; U.S. Pat. Application Publication No. 2004-0086892A1 entitled "UNIVERSAL TAG ASSAY," filed April 24, 2003. Further embodiments are discussed in U.S. Pat. Application Publication No. 2004-0086894A1, entitled "ELECTROCHEMICAL METHOD TO MEASURE DNA ATTACHMENT TO AN ELECTRODE SURFACE IN THE PRESENCE OF MOLECULAR OXYGEN," filed May 2, 2003; U.S. Pat. Application Publication No. 2004-0086895A1, entitled "METHOD OF ELECTROCHEMICAL DETECTION OF SOMATIC CELL MUTATIONS," filed May 2, 2003; and co-pending PCT Application Publication No. W02005/021717A2, filed August 23, 2004.

In particular, U.S. Pat. Application Publication No. 2004-0086895A1 discusses methods of enhancing the signal by elongating the target strand after it has hybridized to the probe strand, a technique sometimes referred to as "on-chip amplification." This application discloses linear as well as branched on chip-amplification.

Other various techniques for detecting nucleic acids, including the use of fluorescein and horseradish peroxidase (HRP), are disclosed in U.S. Pat. No. 6,391,558 (Henkens et al.).

Some preferred embodiments of the present invention include the detection of polynucleotide hybridization in a detection zone. Generally, the detection zone will be located on an assay chip and the detection of hybridization will be carried out by detecting electrical current generated by a redox reaction. An underlying theory of many advantageous embodiments is that the redox reaction only takes place, or takes place to a greater extent, when hybridization between a probe and a target has occurred.

Typically, in carrying out this technique, a plurality of nucleic acid probes which are complementary to a sequence of interest are used. If a universal chip is used, then the nucleic acid probes would contain sequences complementary to a tag attached to a target strand wherein the target strand also contains the sequence of interest. Accordingly, references to detecting a sequence in a sample may refer to directly detecting a natural or native sequence from a biological sample, or may refer to detecting some other sequence (such as a tag sequence) in a processed sample (where the processed sample is made by altering or adding to a sequence derived from a biological sample.)

In certain preferred embodiments, probes range in length from about 10 to 25 base pairs, with a length of about 17 base pairs being most preferred. Preferably, the probe strands are positioned within a detection zone. In particularly preferred embodiments, the detection zone includes a surface, such as an electrode, in contact with a liquid medium, wherein the probe strands are immobilized on the surface such that they are also in contact with the liquid medium. Preferably, the surface is a gold or carbon electrode that is coated with a protein layer such as avidin or streptavidin to facilitate the attachment of the nucleic acid probe strands to the electrode. This protein layer should be porous, such that it allows ions or other electron transfer species to pass from the liquid medium to the electrode and vice versa. When attaching a probe strand to an avidin layer, it is preferable to first bind the probe strand covalently to a biotin complex and then allow the biotin to attach to the avidin. Alternatively, probe strands can be attached directly to the surface, for example by using a thiol linkage to covalently bind nucleic acid to a gold electrode. Carbon electrodes or electrodes of any other suitable conductor can also be used.

In further carrying out this technique, a nucleic acid sample to be interrogated relative to the probe can be contacted with the probe in any suitable manner known to those skilled in the art. For example, the sample may comprise a plurality of target strands. The target strands can be introduced to the liquid medium described above and allowed to intermingle with the immobilized probes; if a target strand contains a region that is complementary to a region of the probe strand, hybridization can take place.

Preferably, the number of target strands exceeds the number of probe strands in order to maximize the opportunity of each probe strand to interact with target strands and participate in hybridization. In some embodiments, however, there will be a relatively small number of target strands; such embodiments include assays in which it is impossible, impractical, or undesirable to isolate or amplify a particular target. This is often the case when detecting the presence of a pathogen, such as a bacterium or virus.

If none of the target strands contains the sequence of interest, there will generally be little or no hybridization between the immobilized probes and the introduced targets. However, if a target strand does contain the sequence of interest (or an appropriate tag when using a universal chip), it may bind to a probe strand. After one or more hybridization events have occurred, there are several techniques that can be used to detect the hybridization.

In some techniques, a moiety capable of participating in a redox reaction can be introduced such that it associates with hybridized DNA by electrostatic attraction. In other techniques, a moiety capable of participating in a redox reaction can be introduced using a sequence-specific detector probe. A detector probe is generally a nucleic acid strand that contains a region that is complementary to a region of the target strand and is coupled to one or more moieties such that an interaction between the detector probe and the moiety capable of participating in a redox reaction is possible. In some preferred embodiments, a detector probe is linked to a fluorescein molecule and the redox moiety is HRP, which is in turn conjugated to a fluorescein antibody capable of binding to the fluorescein. Alternatively, the detector probe can be linked to a biotin moiety, which binds to a streptavidin or avidin HRP conjugate. Further, enzymes other than HRP can be used as a redox moiety. Such enzymes include, for example, glucose oxidase, alkaline phosphatase, and other peroxidases such as thermal stable soybean Peroxidase and microperoxidase.

Further, some embodiments of the present invention can include any combination of the following steps: extracting a biological sample from a patient or an environmental source such that the sample contains or is suspected to contain a first nucleic acid having a particular sequence; purifying or isolating the first nucleic acid from the biological sample; amplifying the first nucleic acid using a technique such as polymerase chain reaction (PCR) or rolling circle amplification (RCA); isolating the first nucleic acid (or its amplification products) in single stranded form; cyclizing a second nucleic acid for the purpose of elongating or otherwise enlarging the first nucleic acid; elongating or otherwise enlarging the second nucleic acid; introducing one or more of the above nucleic acids into an assay environment in which hybridization is possible; controlling hybridization stringency such that some nucleic acids hybridize and others do not based on the degree of complementarity; amplifying one or more of the hybridized nucleic acids on a chip; and detecting hybridization, preferably using a catalytic redox moiety.

It has been discovered that certain techniques can be used to enhance the signal of hybridized nucleic acids. These techniques have been found to be particularly useful in assays in which a small amount of DNA exists in a sample. For example, the present techniques are particularly useful for assays to detect infectious diseases, since the amount of nucleic acid available from the suspected pathogen is typically very small relative to the extracted sample. Particularly useful techniques for enhancing signals of hybridized nucleic acids include catalytic detection and on-chip amplification. Techniques of the present invention can in some cases detect nucleic acids in a sample in quantities as small as 1-10 molecules.

### Catalytic Detection

Some embodiments include the use of catalytic detection moieties instead of counterions (such as ruthenium complexes) which themselves undergo electron transfer at an electrode surface. In a catalytic detection scheme, there are typically two or more moieties able to undergo oxidation/reduction such that at least one of those moieties can participate in more than one redox cycle. Since some of the moieties are in this way "reused," the detectable signal will be larger than if all of the moieties undergo only a single redox cycle. In some embodiments, the catalytic detection moiety is an enzyme which can participate in many oxidation/reduction cycles without being exhausted.

Some preferred embodiments utilize the redox conversion of hydrogen peroxide to water in the electron transfer scheme. In such embodiments, it is advantageous to use a peroxidase such as horseradish peroxidase (HRP) to catalyze this reaction. HRP is a preferred detection reagent because of its stability, high turn over rate, and the availability of sensitive electrochemical mediators. Other enzymes such as phosphatases, other peroxidases including microperoxidase, and oxidases can also be used for this purpose. In particularly preferred embodiments, the electrochemical detection using HRP proceeds as follows:

HRP_{R} + H₂O₂ + 2H⁺ → HRP_{O} + 2H₂O

BIRP_{O} + Med_{R} → HRP_{R}+ Med_{O}

Med_{O} + ne⁻ → Med_{R}

In the reactions above, an R subscript indicates that a species is in its reduced form while an O subscript indicates an oxidized form. In the first step shown above,

In the first step, HRP in its reduced state provides electrons for the reaction of hydrogen peroxide with hydrogen to produce water, thus leaving the HRP in its oxidized state: In the next step, HRP in its oxidized state receives electrons from an electron transfer mediator (denoted as Med) that is able to shuttle electrons from the electrode surface to the enzyme. Preferred mediators include tetramethylbenzidine (TMB) and ferrocene derivatives. In the third step, the transfer mediator receives electrons from the electrode surface. In this particular example, the electrons go from the electrode, to the transfer mediator, to the enzyme, and ultimately end up in a water molecule. The formation of water essentially drives the reaction so that an electron deficit in HRP induces the electron transfer and creates a current through the electrode which can be detected.

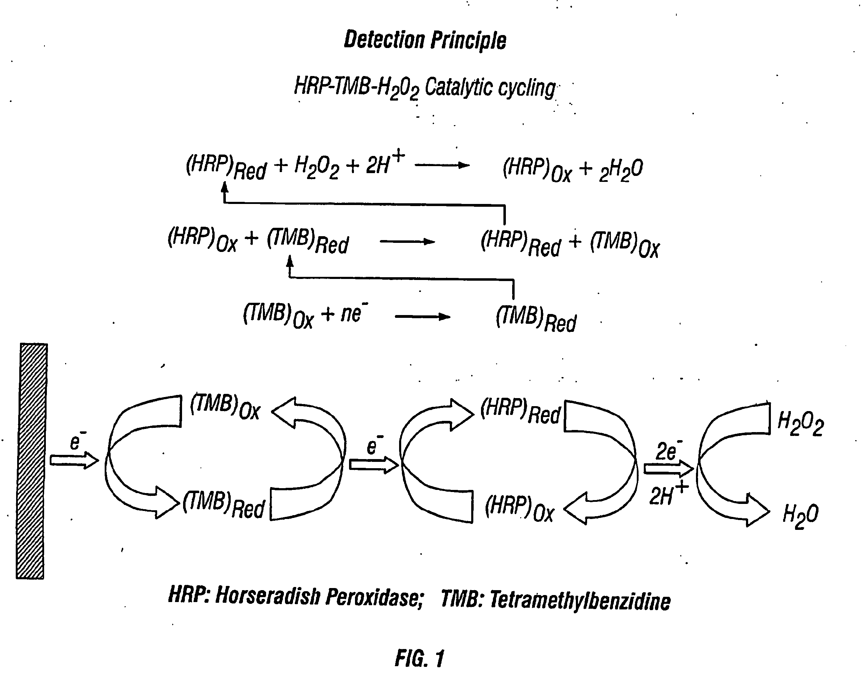

FIG. 1 shows catalytic cycling using HRP and TMB in the conversion of H₂O₂ to water. It will be appreciated by those of skill in the art that other enzymes, particularly other peroxidases can be used instead of HRP, and that other electron transfer mediators can be used instead of TMB.

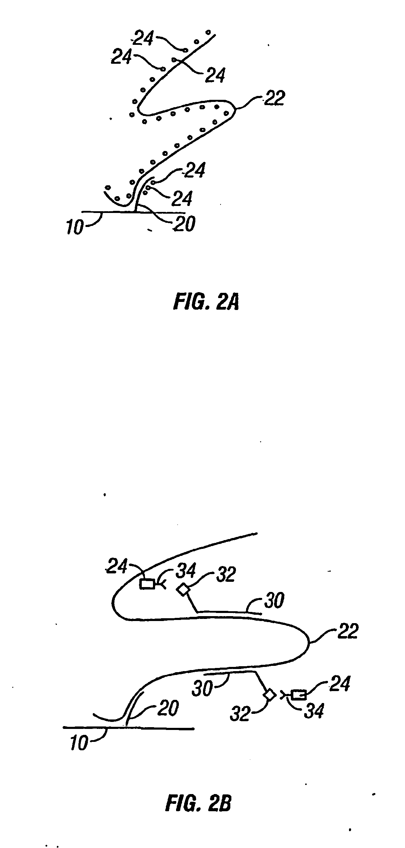

There are two particularly useful techniques (shown in FIGS. 2A and 2B respectively) which can be used to associate an enzyme, such as peroxidase, with a hybridized nucleic acid such that the detectable redox process will serve as a reliable indicator of the hybridization event.

FIG. 2A shows an example of the first of these techniques. As shown, an electrode surface 10 contains an immobilized capture probe 20. A target nucleic acid strand 22 is hybridized to the capture probe 20. A plurality of redox moieties 24 are shown associating with both strands of nucleic acid, the capture probe 20 and the target strand 22. In some embodiments, the redox moiety 24 is an enzyme such as HRP. The redox moiety can also be an enzyme conjugate, which means that an enzyme is conjugated to another species before being introduced to the assay for association with the nucleic acids. In certain advantageous embodiments, an enzyme is conjugated to a polyamine, a cationic peptide, or a cationic polymer. Polyamines such as spermidine or spermine can be conjugated to HRP through various conjugation schemes including periodate coupling chemistry or glutaraldehyde mediated conjugation. Useful cationic peptides include polylysine and polyarginine. Cationic polymers, such as those containing an amine group can also be used. In some embodiments, conjugation is accomplished using an EDC-facilitated conjugation, which is described, for example, in Hermanson, Greg T., Bioconjugate Techniques, 1996 Academic Press San Diego, pp. 420-435. Various other techniques for placing a cationic charge on the enzyme or on a moiety coupled to the enzyme are known in the art and can also be used.

The cationic enzyme conjugate can then associate electrostatically with the negatively charged phosphate backbones of the target. Then, a reaction catalyzed by the enzyme can generate an electrical current which can be measured as an indication of the presence of the target nucleic acid in the sample. In some preferred embodiments, the enzyme is HRP and the reaction is that of hydrogen peroxide with hydrogen to produce water. In some preferred embodiments, a DNA or RNA target is first hybridized to a capture probe that has been immobilized onto an electrode surface. Polyamine-HRP conjugate is then provided in solution to bind to the target. After washing the unbound HRP, the electrode is connected to an electrochemical monitor. At fixed potential a strong catalytic current is produced from the surface immobilized HRP in the presence of sufficient concentrations of both H₂O₂ and a mediator. The generated current is proportional to the amount of surface immobilized HRP, and consequently to the number of nucleotide bases in the target.

As an alternative, FIG. 2B shows an example of a second detection method. Here, an electrode surface 10 contains an immobilized capture probe 20 with a hybridized target strand 22. An additional nucleic acid detector probe 30 hybridizes to the target strand 22 (preferably in an amplified region of the target strand). In some embodiments, the target strand from the sample is hybridized directly to the capture probe. In other embodiments, the target strand in the sample is amplified and the resulting amplification products are hybridized to the capture probe. The detector probe 30 is in turn labeled with a hapten 32 to which an antibody 34 linked to a redox moiety 24 is able to bind. Numerous suitable haptens can be used; examples of preferred haptens are fluorescein, digoxigenin, or biotin, wherein the corresponding antibody would be a fluorescein, digoxigenin, or biotin antibody respectively. Alternatively, if biotin is used as a hapten, avidin or an avidin analog can be used as a substitute for an antibody because of its binding affinity for biotin. It is advantageous that the antibody 32 be linked to an appropriate redox moiety 24, such as an enzyme like HRP. In some embodiments, the antibody can be linked to an avidin-HRP construct. It is generally advantageous to use multiple detector probes that can bind to a single target. Detection of hybridization is again based on the catalytic current due to the presence of a redox moiety, such as HRP. The current is proportional to the amount of redox-moiety present which is in turn proportional to the number of detector probes hybridized to the captured target. In some embodiments, each detector probe can contain multiple sites at which enzyme-antibody complexes can attach, further enhancing the electrical signal.

Catalytic detection of this type generally exhibits various advantages. For example, the sensitivity and detection limit can be significantly improved for quantitative analysis. Unlike most other detection methods where each target molecule is labeled with one detection reagent, the detection of multiple labels per target molecule by a sensitive enzyme catalyzed electrochemical reaction is possible.

Further, some embodiments of the present invention feature a lower detection noise level than previous techniques. If uncharged capture probes such as methyl phosphonate DNA or PNA are used, the current detection range can be about 0 nano amp to 100 micro amps. Uncharged capture probes are discussed in greater detail in co-pending U.S. Application Publication Nos. 2004-0086894A1 and 2004-0086895A1.

Additionally, the reaction time can be shortened and assays can be made less complex using catalytic detection techniques.

### On-chip Amplification

Another technique for enhancing the signal of a nucleic acid duplex on a substrate such as a chip is to amplify or extend the target nucleic acid after hybridization to the capture probe. The amplified or extended target nucleic acid can then be bound to a detection reagent. Preferably, the target strand is elongated so as to make the difference between the current at an unhybridized capture probe and the current at a probe/target duplex as profound as possible. This technique may be referred to as "on-chip amplification" and is disclosed in copending U.S. Pat. Application Publication No. 2004-0086895A1.

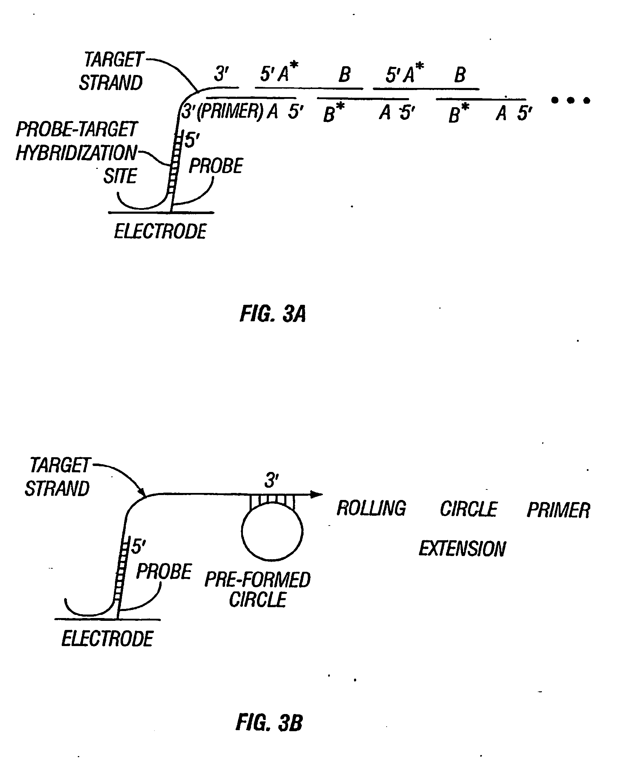

Various methods of on-chip amplification can be used. One method of on-chip amplification is depicted in FIG. 3A. Here, either the 3' or 5' end of a hybridized target strand can be targeted for a head-to-tail polymerization that builds up the amount of DNA on the electrodes. Typically, three different oligonucleotides (not counting the immobilized probe and the target strands) will be used as shown here: the first oligomer is complementary to the 3' end of the hybridized target strand (targeting the complement of the primer sequence), and contains a sequence A at its 5' end; the second oligomer has a sequence 5'-A*B-3', where A* is complementary to A; the third oligonucleotide has sequence 5'-AB*-3'. As depicted in FIG. 3A, these oligomers can form a polymeric product as shown. The head-to-tail polymerization can continue until the strand reaches a desired length. Generally, when performing head-to-tail polymerization, the ultimate length of the polynucleotide is limited in part by a competing cyclization reaction of the head-to-tail oligomers. A higher concentration of head-to-tail oligomers in the liquid medium will generally produce longer linear polymers attached to the electrode, however.

A second method of on-chip amplification is depicted in FIG. 3B. This method uses rolling circle amplification (RCA). Preferably, a preformed circle (approximately 40 to 300 nucleotides) that has a region complementary to the 3' end of the bound target nucleic acid from the sample, or an amplification or elongation product produced therefrom, is hybridized to the target nucleic acid or amplification or elongation product produced therefrom which is hybridized to the capture probe. A processive DNA polymerase can then be added so that RCA results, elongating the bound target nucleic acid from the sample or amplification or elongation product produced therefrom. Preferably, the target nucleic acid from the sample is elongated by approximately 10 to 10,000 copies , of the circle. The detection reagent is then bound to the RCA product.

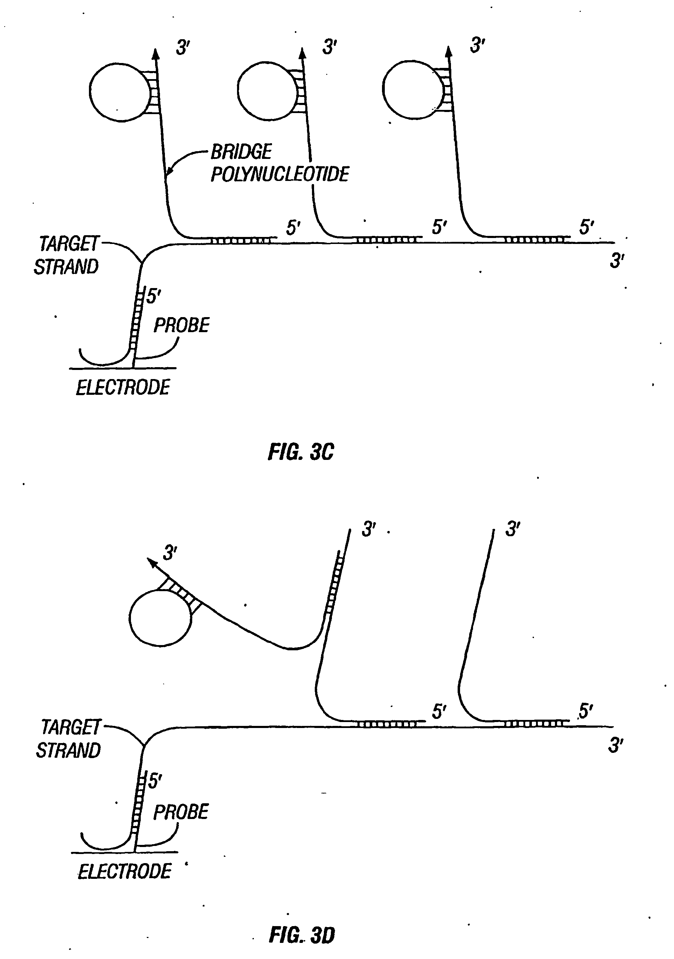

A further technique for on-chip amplification is depicted in FIG. 3C. This technique may be referred to as "bridge" amplification or "branch" amplification, as the terms "bridging" and "branching" are synonymous in this context. In this method, the target nucleic acid from the sample or an amplification or elongation product produced therefrom is hybridized to the capture probe. A first preformed circle is hybridized to the target nucleic acid from the sample or an amplification or elongation product produced therefrom, and RCA is performed to produce a first RCA product. A bridge nucleic acid comprising a sequence complementary to a sequence in the first RCA product and a sequence complementary to a sequence in a second preformed circle is provided. The bridge nucleic acid is hybridized to the first RCA product and a second RCA procedure is performed with the second preformed circle, thereby producing a second RCA product. The detection reagent is then bound to the target nucleic acid from the sample or amplification product or elongation product produced therefrom, first RCA product, bridge nucleic acid, second RCA product, or any two or more of the preceding nucleic acids. It will be appreciated that the first RCA procedure and/or the second RCA procedure may be substituted with head-to-tail polymerization or with another elongation procedure if desired. It will also be appreciated that rather than using a preformed circle for rolling circle amplification, a linear molecule containing sequences at each end which hybridize to the target nucleic acid or amplification or elongation product produced therefrom or to the bridge nucleic acid may be circularized after hybridization to generate a circular template for RCA.

Further, when a bridge amplification technique is used, it can be advantageous to increase the extent of branching using a technique known as "hyperbridging" or "hyperbranching" An example of hyperbridging is shown in FIG. 3D. Here, a second bridge nucleic acid comprising a sequence complementary to a sequence in the second RCA product and a sequence complementary to a sequence in a third preformed circle is provided. The second bridge nucleic acid is hybridized to the second RCA product and a third RCA procedure is performed with the third preformed circle, thereby producing a third RCA product. The detection reagent is then bound to the target nucleic acid from the sample or amplification or elongation product produced therefrom, first RCA product, first bridge nucleic acid, second RCA product, second bridge nucleic acid, third RCA product, or any two or more of the preceding nucleic acids. It will be appreciated that the first RCA procedure, second RCA procedure, third RCA procedure, or any combination thereof may be substituted with head-to-tail polymerization or with another elongation procedure if desired. It will also be appreciated that rather than using preformed circles in any of the RCA procedures, a linear molecule containing sequences at each end which hybridize to the target nucleic acid or amplification or elongation product produced therefrom to the first bridge nucleic acid, or to the second bridge nucleic acid may be circularized after hybridization to generate a circular template for RCA.

It is generally advantageous to use an elongation technique such as rolling circle amplification or head-to-tail polymerization in conjunction with a hyperbridging process. Elongation generally serves the purpose of adding nucleic acid material that can be detected electrochemically to help distinguish hybridized from unhybridized nucleic acids, but the process can also be beneficial since longer nucleic acids provide more locations in which additional bridges can be attached.

Bridging and hyperbridging can be particularly useful techniques since the amount of nucleic acid present can be increased exponentially. Additional discussion of bridging and hyperbridging techniques can be found, for example, in: Urdea, Biotechnology 12:926 (1994); Horn et al., Nucleic Acids Res. 25(23):4835-4841 (1997); Lizardi et al., Nature Genetics 19, 225-232 (1998); Kingsmore et al. (U.S. Pat. No. 6,291,187); Lizardi et al. (PCT application WO 97/19193).

When performing an assay that includes on-chip amplification, it is possible to detect hybridization with or without using a bridging step. Assuming that a detector probe is used to associate HRP with hybridized nucleic acid, the following two examples outline steps that could be taken in an assay that does not feature bridging, and an assay that does feature bridging, respectively.

A method without bridging generally includes the following steps: 1) bind the target to the probe; 2) (optional) determine specificity through ligation; 3) perform RCA on the bound target; 4) add a detector probe containing an epitope, such as fluorescein; add an antibody linked to a redox moiety such that the antibody is capable of binding to the epitope; 5) detect the redox activity related to the presence of the redox moiety.

A method that features a bridging step generally includes the following: 1) bind the target to the probe; 2) (optional) determine specificity through ligation; 3) perform RCA on the bound target; 4) add a bridge nucleic acid and a second circle; 5) perform RCA at the bridge; 6) add a detector probe which hybridizes to the RCA product containing an epitope, such as fluorescein; 7) add an antibody linked to a redox moiety such that the antibody is capable of binding to the epitope; 8) Detect the redox activity related to the presence of the redox moiety.

After performing an on-chip amplification, the increased amount of DNA can generate a larger and more detectable signal. This can be advantageous for assay purposes since both the probe and the target typically produce some detectable signal. If the signal of the target is enhanced, the contrast between hybridized and unhybridized capture probes will be more profound. In some embodiments, however, nucleic acid analogs such as methyl phosphonates and PNAs can be used as capture probes. In other embodiments, nucleic acid analogs such as methyl phosphonates and PNAs can be used as detector probes

Accordingly, on-chip amplification of nucleic acid is one useful technique for enhancing the signal of hybridized target. On-chip amplification can be used alone, or in conjunction with other signal enhancing techniques such as catalytic detection. Accordingly, some preferred embodiments include the steps of: (1) hybridizing a target to a capture probe immobilized on an electrode surface; (2) performing on-chip rolling circle amplification using a polymerase with high processivity and strand displacement capability; (3) binding a detector probe to the amplified product on the chip; and (4) detecting catalytic signal generated from a highly electrochemically reactive enzyme.

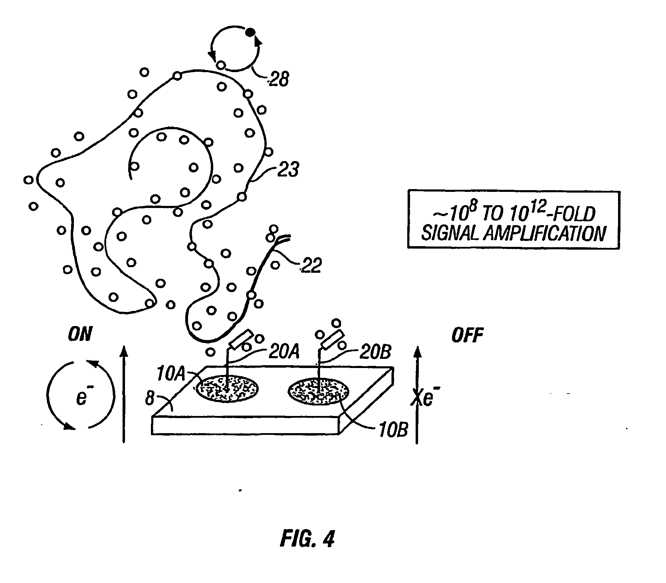

FIG. 4 illustrates signal amplification for direct pathogen detection using both on-chip amplification and catalytic detection with HRP. Two electrode surfaces, 10A and 10B are shown positioned on a substrate 8. Capture probes 20A and 20B (having different sequences) are immobilized on the electrode surfaces 10A and 10B respectively. A target strand 22 is introduced to the assay. This target 22 is complementary to capture probe 20A, but not to capture probe 20B. Accordingly, target 22 hybridizes to capture probe 20A, but not 20B. Once the target 22 is hybridized, it is elongated by on-chip amplification so that the elongated portion may be referred to as the on-chip RCA product 23. A plurality of redox moieties 24 are then introduced to the assay. These moieties contain a cationic charge and associate electrostatically with the phosphate groups of the nucleic acids. The redox moieties associate with the capture probes 20A and 20B, target strand 22, and RCA product 23. A plurality of redox reactions 28 occurs at each of the redox moieties. These redox reactions include a transfer of electrons, which generates a current as electrons pass through the electrodes 10A and 10B. Because the quantity of reactions at the 10A electrode exceeds the quantity of reactions at the 10B electrode, the detectable electrical signal at the 10A electrode is greater, which means that a user of the assay can conclude that the 20A capture probe is complementary to the target 22 and that capture probe 20B is not.

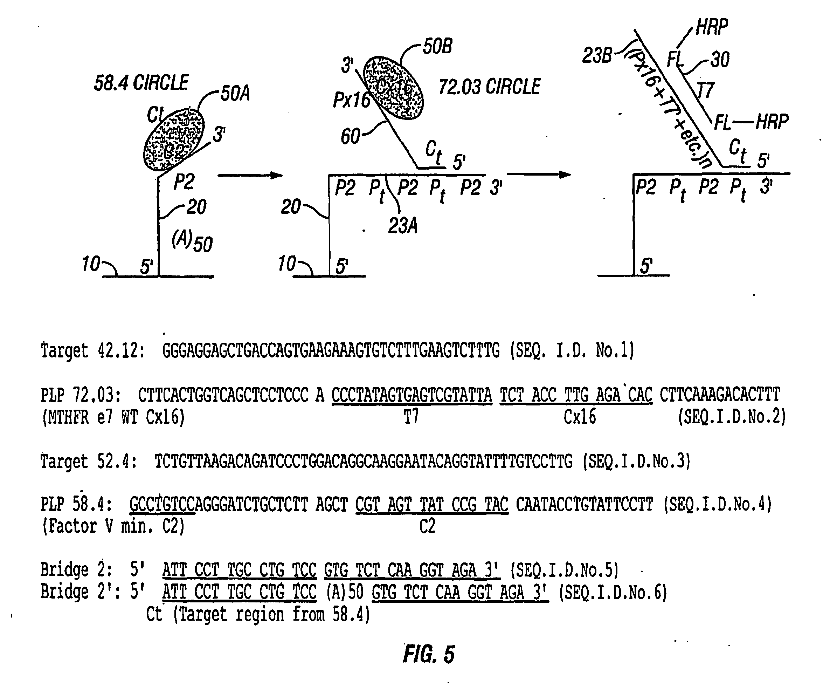

FIG. 5 shows a scheme for RCA and signal bonding. In this illustration, branching (also known as "bridging") is used to further augment the DNA present and enhance the signal. Here, a capture probe 10 is immobilized on an electrode surface 10. A first circle 50 is then hybridized to the probe 20 (in the area where "P2" on the capture probe 10 is complementary to "C2" on the circle 50.) After hybridization, RCA proceeds, generating a first RCA product 23A that extends from the immobilized capture probe 20. Next, a bridge 60 is hybridized to the first RCA product 23A (the "Pₜ" region of the first RCA product 23A is complementary to the "Ct" region of the bridge 60.) Then, a second circle 50B hyridizes to the bridge 60 (the "Px16" region of the bridge 60 is complementary to the "Cx16" region of the second circle 50B. RCA then proceeds where the second circle 50B is hybridized to the bridge 60, generating a second RCA product 23B. Then, a detector probe 30 hybridizes to the second RCA product 23B. Fluorescein (FL) is shown attached to detector probe 30 and HRP is in turn coupled to the fluorescein (by an antibody complex, for example). The HRP can then participate in a redox reaction to induce an electron flow which is detectable at the electrode 10. Nucleic acid sequences are also provided.

In some embodiments, signal amplification by RCA can be recorded using Osteryoung Square Wave Voltammetry (OSWV). Using this technique, it was observed that a non-circle control generated a current of about 0.050 µA at a voltage of -200 mV (in a "non-circle" control, RCA does not take place after hybridization). A non-complimentary control capture probe (sequence 65.5) was also observed to produce a current of about 0.050 µA at a voltage of -200 mV (in a "non-complementary" control the target is not complementary to the capture probe, and therefore does not hybridize). A target that hybridized and underwent amplification by RCA, however, generated a current of about 0.450 µA at a voltage of -200 mV.

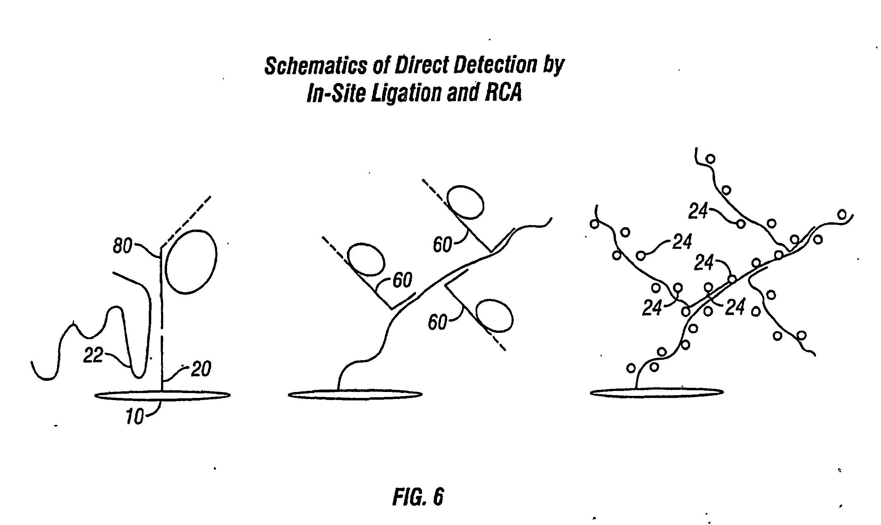

FIG. 6 shows an example of an alternative elongation method using an in-situ ligation of the immobilized probe. As shown, a capture probe 20 is immobilized on an electrode 10 and a complementary target 22 having a region extending beyond the free end of the immobilized probe is allowed to hybridize to the capture probe 20. Then, a third polynucleotide 80 having a region complementary to the section of the target 22 that extends beyond the capture probe 30 is allowed to hybridize to the hybridized target 22. Next, the third polynucleotide 80 is ligated to the immobilized capture probe 30. To accomplish this, the third polynucleotide 80 may hybridize to a sequence in the target 22 which is immediately adjacent to the region in which the target 22 is complementary to the probe 20. Alternatively, there may be a gap between the target sequence complementary to the probe 20 and the target sequence complementary to the third polynucleotide 80. In embodiments where there is a gap, the gap may be filled in using a polymerase prior to ligation. Advantageously, the third polynucleotide 80 is extended, for example by rolling circle amplification. It is also advantageous in many embodiments to hybridize bridges 60 to the immobilized nucleic acid. Elongation of those bridges further enhances the amount of nucleic acid immobilized on the electrode. As shown in FIG. 6, a redox moiety 24 such as a transition metal complex (such as ruthenium complex) or an enzyme (such as HRP) can then associate with the nucleic acid as an indicator of the initial probe/target hybridization.

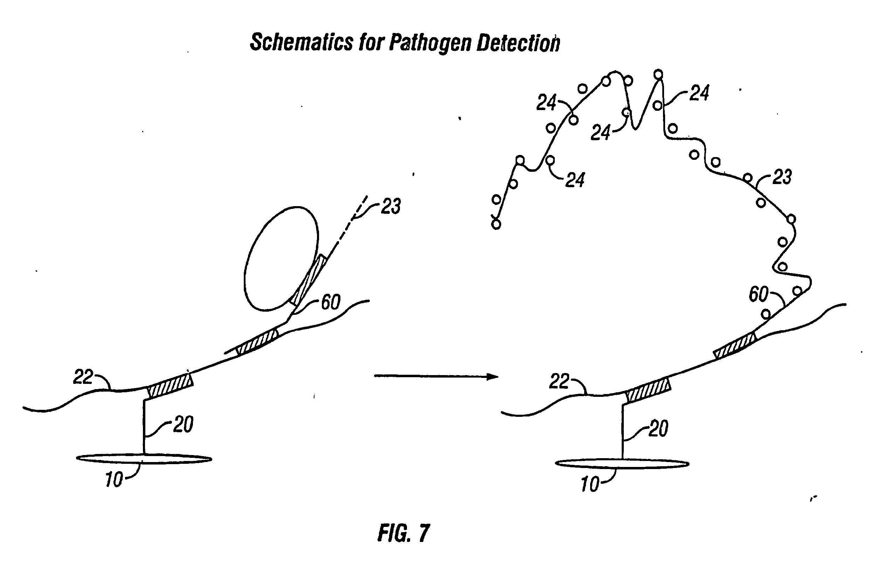

FIG. 7 shows an example of pathogen detection wherein a target nucleotide 22 from a pathogen hybridizes to an immobilized probe 20, a bridge 60 is attached to the target 22, and the bridge 60 is elongated by rolling circle amplification to create an RCA product 23. In this example, redox moieties 24 associate only with the bridge 60 and the RCA product 23. This is advantageous in pathogen detection since the amount of target that is available to be detected is often very small. Preparing the assay in a manner that prevents the redox moieties 24 from associating with the probe 20 and the target 22 can be achieved by using nucleic acid analogs such as phosphonates and PNAs that don't contain a charged backbone. This technique can be used to minimize background noise caused by electron transfer at electrodes where the nucleic acid hybridizations have not occurred, thus making a true positive result that much more profound by comparison.

### Examples

### Example 1: Nucleic acid assay using on-chip amplification, bridging, and catalytic detection

The following process was used in a nucleic acid detection assay.

Biotinylated capture probes were immobilized on an electrode surface which was modified with NeutrAvidin (a deglycosylated form of avidin, available from Pierce Biotechnology, Inc.). The immobilized capture probes also served as the primer for DNA extension.

A circular 58 mer DNA (circle 1) was hybridized to the capture probe immobilized on the surface of the electrode. The primer extension by rolling circle amplification (RCA) was initiated in the presence of Bst DNA polymerase and other necessary chemicals. Operation of RCA converted dNTPs into a single-stranded concatameric DNA molecule composed of thousands of repeated copies of the circle. The quantity of circles used was very low, in the range of hundreds to thousands of DNA molecules.

Another DNA molecule bridging between the RCA product in Step 2 and a second circular DNA (circle 2) was hybridized to concatameric DNA molecule generated in Step 2. The second circular DNA was then hybridized to the bridge DNA molecule. RCA reaction was performed again. This second RCA reaction resulted in a branched DNA; accordingly, signal amplification by means of successive RCAs is exponential.

A 15 mer DNA molecule complementary to a portion of the branched DNA produced by RCAs and modified with two fluorescein molecules was hybridized to the branched DNA. An anti-fluorescein-POD was then bonded to the 15 mer fluorescein-modified DNA to serve as the detector probe.

Detection of circle 1 was carried out by catalytic reduction of H₂O₂ to water by POD with TMB as the mediator. The readout is a steady-state current-time curve. The current response in the presence of very low circle 1 concentration was several times more than that in the absence of the circle.

### Example 2: Alternative nucleic acid assay using on-chip amplification, bridging, and catalytic detection

A capture probe immobilized through avidin immobilized on an electrode surface contains a sequence complementary to a target. Sample nucleic acids are then hybridized to the probe. A circular probe which contains a sequence complementary to a sequence within the target is added. The circle also contains a specific sequence which is complimentary to a detector probe to be used in later steps.

The circle, preferably with a size smaller than 100nt, first binds to the target nucleic acid which is hybridized to the probe immobilized on the electrode. In the presence of a polymerase enzyme and with the target nucleic acid as a primer, the circle sequence is copied multiple times at a constant temperature, resulting in surface immobilized elongated DNA molecules which contain multiple repeats complementary to the sequence of the circle including multiple copies of the sequence complementary to the detector probe. The preferred DNA polymerases for on-chip RCA include those having high processivity and strong strand displacement features such as Φ29 DNA polymerase, Bst DNA polymerase, large (Klenow) fragment of E.coli DNA polymerase, and sequenase.

After washing, the elongated DNA product is hybridized to a hapten-labeled detector probe complementary to a portion of the RCA product. The hapten such as fluorescein or Digoxiginin subsequently binds to an antibody-HRP conjugate and in the presence of an electron transfer mediator and hydrogen peroxide generates catalytic current through an enzymatic electron transfer reaction.

Alternatively, rather than hybridizing the detector probe to the RCA product as described above, a second RCA product is generated as follows. In this embodiment, linear bridge nucleic acid comprising a nucleotide sequence complementary to a portion of the RCA product and a nucleic acid sequence complementary to a second circular probe is hybridized to the RCA products such that the bridge nucleic acid bridges the amplification product from the first circle through its 5' end and the second circle through its 3' end. During RCA, the first circle generates linear DNA products containing multiple repeats of complementary sequence of the first circle, while the second circle generates a second RCA product. As a result, millions to billions-fold amplification can be achieved at a constant temperature in hours, allowing sensitive detection of DNA targets. After amplification a detector probe is allowed to bind to the repeat sequence present in DNA products generated from both circles. Finally, an antiFI-HRP conjugate binds to the detector probe and further amplifies the signal through catalytic electrochemical detection.

The detector probes can also be added in the amplification solution to simplify the procedure.

### Example 3: Use of a Padlock Probe Circle

A padlock probe circle (PLPC) was used along with an anti-fluorescein-HRP conjugate in the following nucleic acid detection assay.

A 250 nM sample of FV-PLP 75.03 (PLPC, SEQ. I.D. No. 13) was used to prepare dilutions of 1:100, 1:1000, and 1:10,000. Capture probes (65.4) (SEQ. I.D. No. 26) were immobilized on a chip and circles from each dilution were then bound to the capture probes at 37 °C for 15 minutes. An on-chip RCA reaction utilizing the bound circles then proceeded at 60 °C for one hour.

The immobilized nucleic acids were then incubated with 0.25 µM FL-T7-FL signal probe in hyb buffer at room temperature for 20 minutes. A 1:200 dilution of anti-fluorescein-HRP in PBS/casein was incubated with the chips at room temperature for 20 minutes. Steady-state current measurements were taken for 30 seconds in Enhanced K-Blue solution (obtained from Neogen Corp.).

Table 1 shows the results. As shown, the number of molecules of PLPC per electrode was observed to have a direct relationship with the detectable current at the electrode.

**Table 1**

| Dilution | PLPC Concentration | # Molecules/electrode | Steady State Current, µA |
|---|---|---|---|
| 1:100 | 2.5 nM | 4 x 10⁹ | 1.079 |
| 1:1000 | 250 pM | 4 x 10⁸ | 0.730 |
| 1:10,000 | 25 pM | 4 x 10⁷ | 0.131 |
| No PLPC | 0 | 0 | 0.016 |

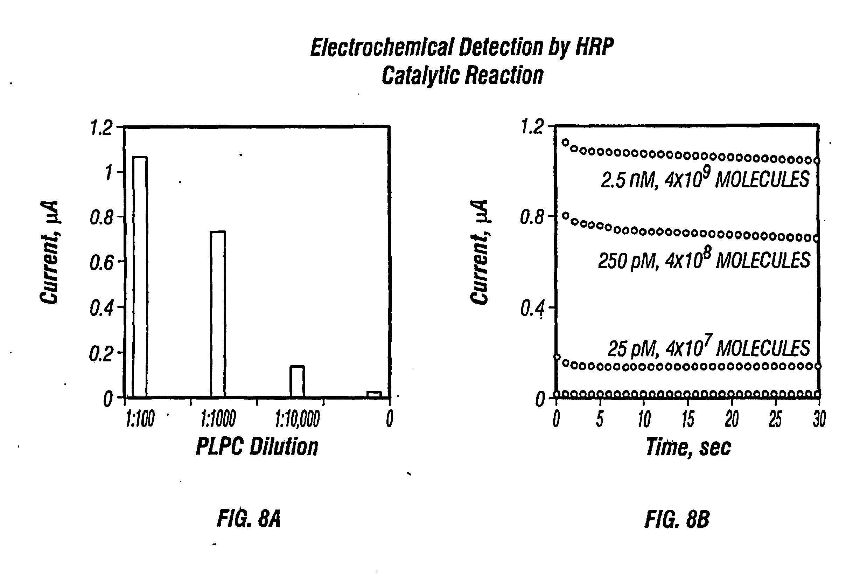

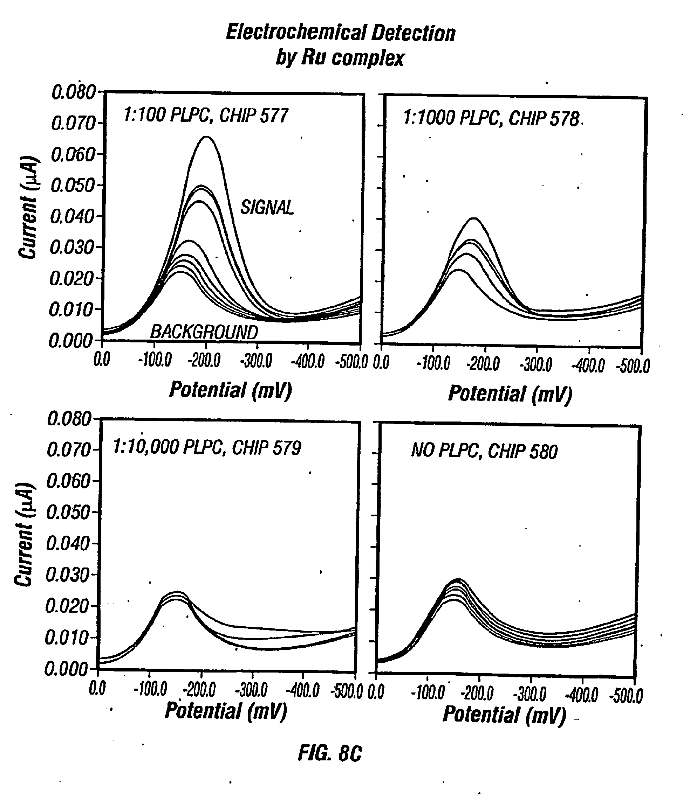

Detection using the HRP catalytic method was compared to ruthenium complex electrostatic detection. Detection using a ruthenium complex, such as ruthenium hexamine, for example, is not a catalytic process, but instead deposits transferred electrons in the ruthenium complex, where they remain, meaning that a reduced ruthenium complex would not be reused to receive more electrons. Hence, a process that is non-catalytic in this way is limited in its precision by the quantity of redox moieties (such as ruthenium hexamine) that are used. FIG. 8 shows the results of the comparison of catalytic detection to non-catalytic detection. Catalytic detection is shown as a bar graph with a corresponding time course at the top of the figure. The non-catalytic detection using the ruthenium complex is shown in the four line graphs at the bottom. In the ruthenium results, twelve signals for each graph correspond to three readings on a four-pad chip. The lowest set of four signals is NeutrAvidin alone, the next four represent NeutrAvidin plus the capture probe, and the highest four represent NeutrAvidin plus the capture probe plus the RCA product of a captured amplicon.

As shown in FIG. 8, the catalytic detection allows greater sensitivity, particularly in very dilute samples. For example, in the catalytic detection method, the dilutions of 1:100, 1:1000, 1:10,000, and 0 are all easily distinguished from each other based on the corresponding current. In the ruthenium detection, however, the dilutions of 1:100 and 1:1000 are easily distinguished, but there is no perceptible difference between the current corresponding to the dilutions of 1:10,000 and 0. Accordingly, there is a detection limit somewhere between the 1:1000 and 1:10,000 dilutions when using ruthenium whereas the detection limit when using HRP is considerably lower.

### Example 4: Quantitative Detection

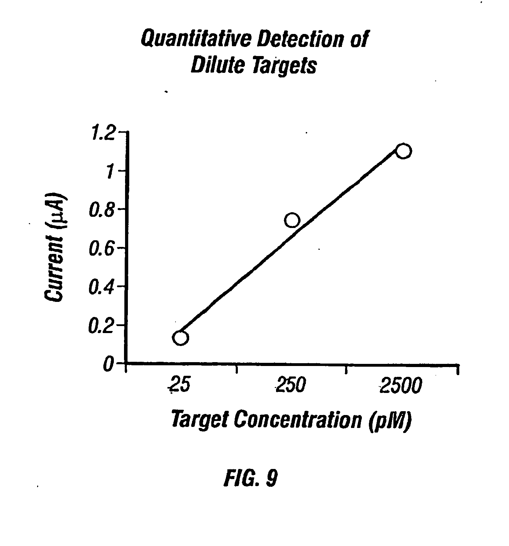

FIG. 9 shows a further application of the present invention in which target nucleic acid in a sample can be quantified. As shown, target nucleic acid in concentrations of 25 pM, 250 pM, and 2500 pM were detected using a catalytic process. The detectable current varied with the concentration of target nucleic acid present which would allow a prediction of target nucleic acid concentration in a sample based on detected current wherein the actual concentration is unknown. Practical applications of this embodiment can include, for example, determining the prevalence of a pathogen in a particular sample. More specifically, this technique could be used to determine the viral load in a patient suffering from an infection by a particular virus.

### Example 5: Detection of Hybridization Using On-Chip Amplification and Catalytic Detection While Varying The Concentrations of Targets, Bridges, and Circles

The following process was used in a hybridization detection assay.

A film for deposition on an electrode surface was prepared using the components listed in Table 2.

**Table 2**

| Capture Probe Seq. | 26.10 (SEQ. I.D. No. 21) |
|---|---|
| NA | 24 µl |
| Tris EDTA buffer | 7.2 µl |
| 10 mM Hepes + 50 mM LiCl | 40 µl |
| Capture Probe (probe 26.10 @ 100 µM) | 7.2 µl |
| H₂O | 26.6 µl |
| Iso-propyl alcohol | 15 µl |
| Vₜₒₜₐₗ | 120 µl |

2 µl of this solution was transferred to various electrode spots on gene chips and allowed to dry. Stable coat was applied and vacuum dried. The dried films were stored in a desiccator overnight.

The dried films were placed in 10 mM Hepes + 250 mM LiCl + 0.05 % Tween 20 @ 37°C for 15 minutes, followed by a rinse in 10 mM Hepes + 10 mM NaCl.

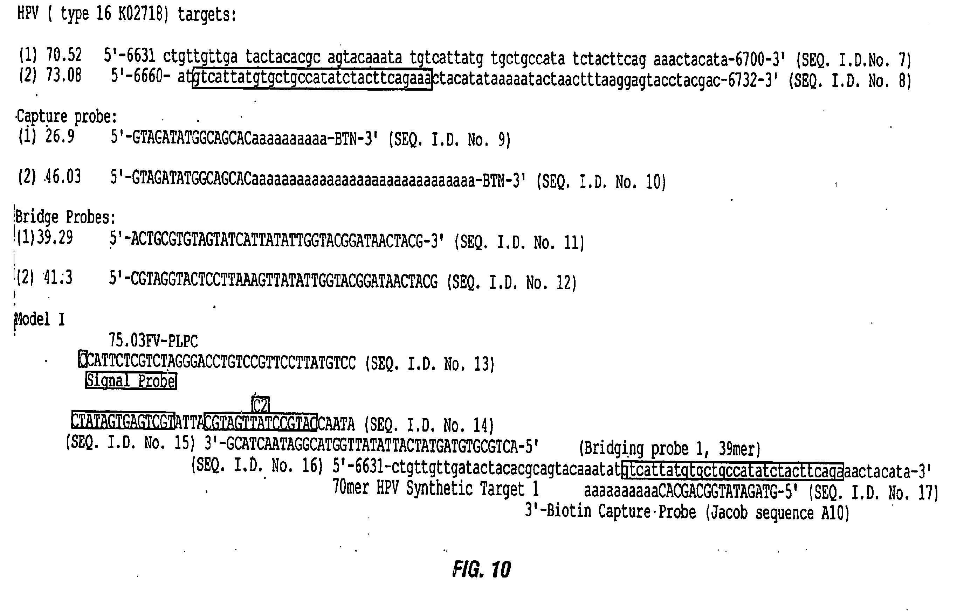

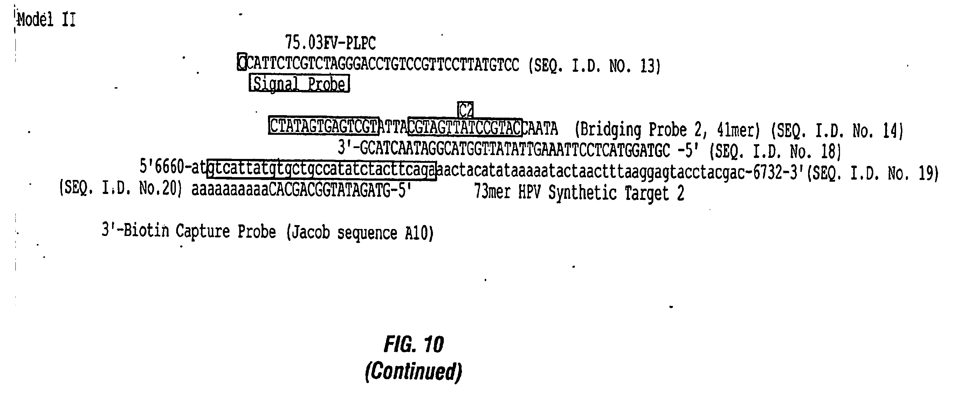

Sequences used in this assay are shown in FIG. 10. Hybridization of the target (70.52) (SEQ. I.D. No. 7), bridge (39.29) (SEQ. I.D. No. 11), and circle (75.03) (SEQ. I.D. No. 13) was performed in solution by mixing 2.5 nM of each in 10 mM Hepes + 1 M LiCl or 10 mM Tris + 1 M NaCl + 1 mM EDTA. The mixture was heated up to 80 °C for 10 minutes and cooled to room temperature.

Hybridization of above mixture to the capture probe was accomplished by applying 10 µl of the mixture to each chip surface and incubating at 37 °C for 15 minutes and cooling to room temperature on the lab bench.

After hybridization, the solutions on the chip surfaces were removed with blotting paper followed by a wash with 10 mM Hepes + 1 M LiCl or 10 mM Tris + 1 M NaCl at pH, 7.5.

The electrodes were dried with the blotting paper and 20 µl RCA working solution were transferred to the surface of each chip. RCA was carried out at 37°C for 1 hour. The 10 µl RCA working solution contained 1 µl 10X buffer, 1.5 µl 10 mM dNTP, 0.5 µl 2 M KCl, 6.5 µl water and 0.5 µl phi29 DNA polymerase.

The RCA working solution which remained on the surfaces of the chips was dried with the blotting paper, followed by rinse with 10 mM Tris + 200 mM NaCl and stored in 10 mM Tris + 200 mM NaCl.

Hybridization with the detection probe (part of T7 sequence modified with two Fluoresceins) was then performed. To accomplish this, 0.25 µM of detection probe in 10 mM Tris + 1 M NaCl + 1 mM EDTA + 0.05 % BSA was provided in the hybridization solution. Hybridization was at 37°C for 15 minutes and cooled to RT on the lab bench.

The chips were dried with blotting paper and rinsed with 1XPBS (0.008 M NaPi + 0.002 M Kpi + 0.14 M NaCl + 0.01 M KCl pH 7.4)

The PBS solution which remained on the surfaces of the chips was removed with the blotting paper. The chips were, then, applied to the hybridization well containing 1:200 dilution of Anti-fluorescein-POD in PBS containing 0.5% casein.

The chips were dried with blotting paper followed by a rinse with 1XPBS and stored in 1XPBS.

K-blue solution was used as received. The measurements were carried out at -200 mV vs. Ag/AgCl (Ag wire) for 30 seconds.

In preparing the solutions to be deposited on the chips, two concentrations of target nucleic acid were used and four concentrations of bridge and circle nucleic acids were used. The concentrations are shown in Table 3.

**Table 3**

| | 1 | 2 | 3 | 4 |
|---|---|---|---|---|
| [Target] | 2.5 e⁻³ nM, (approximately 5 e⁶ molecules) | | | |
| [Bridge] | 0.2 nM | 0.1 nM | 0.05 nM | 0.025 nM |
| [Circle] | 0.24 nM | 0.12 nM | 0.06 nM | 0.03 nM |
| | | | | |
| [Target] | 2.5 e⁻⁴ nM, (approximately 5 e⁵ molecules) | | | |
| [Bridge] | 0.2 nM | 0.1 nM | 0.05 nM | 0.025 nM |
| [Circle] | 0.24 nM | 0.12 nM | 0.06 nM | 0.03 nM |

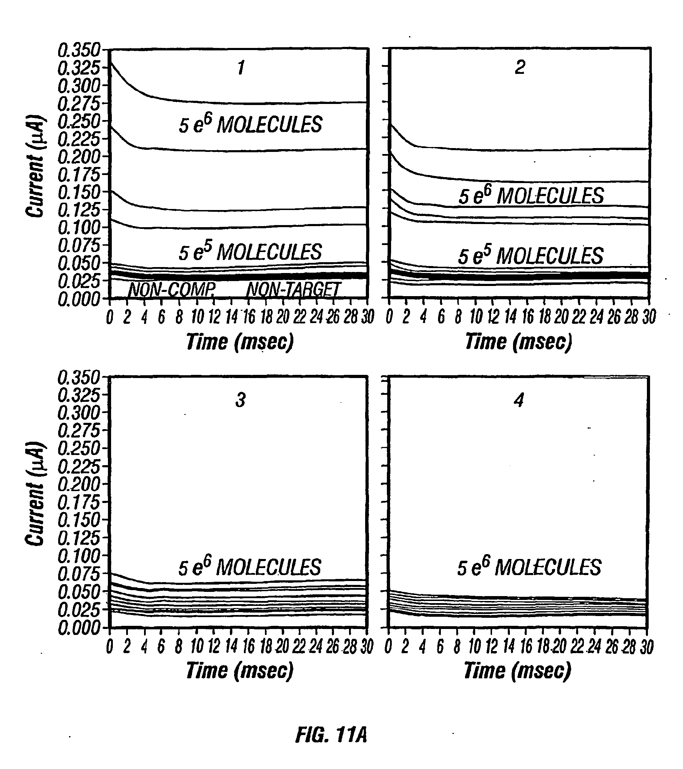

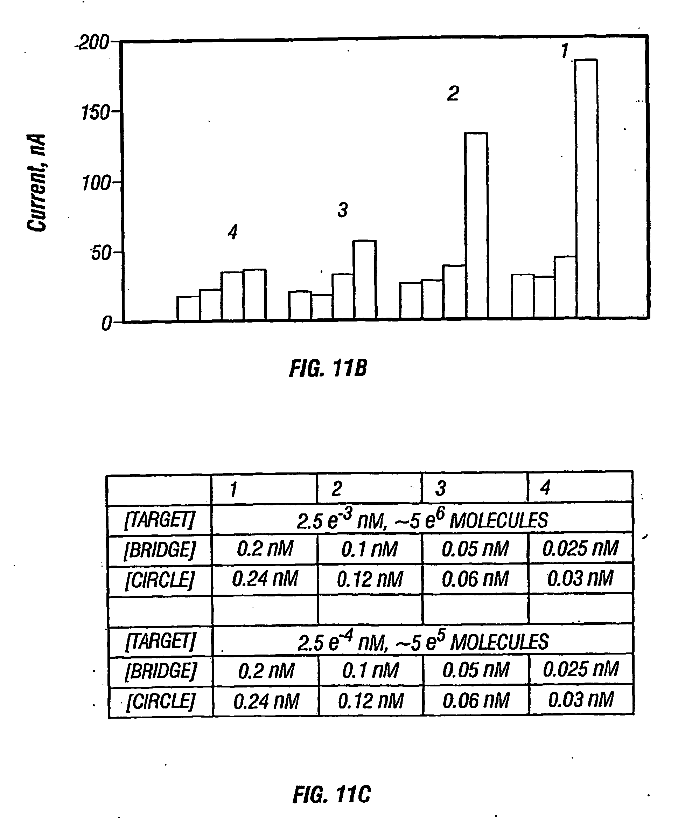

The results of the assay are shown in FIG. 11. As depicted, varying the concentrations of the target, bridge, and circle nucleic acid strands resulted in current levels which correlated with the concentration of the target nucleic acid, thereby demonstrating the effectiveness of the foregoing methods in quantitating the level of target nucleic acid present in a sample.

### Example 6: Two-Stage RCA with Detection Using HRP

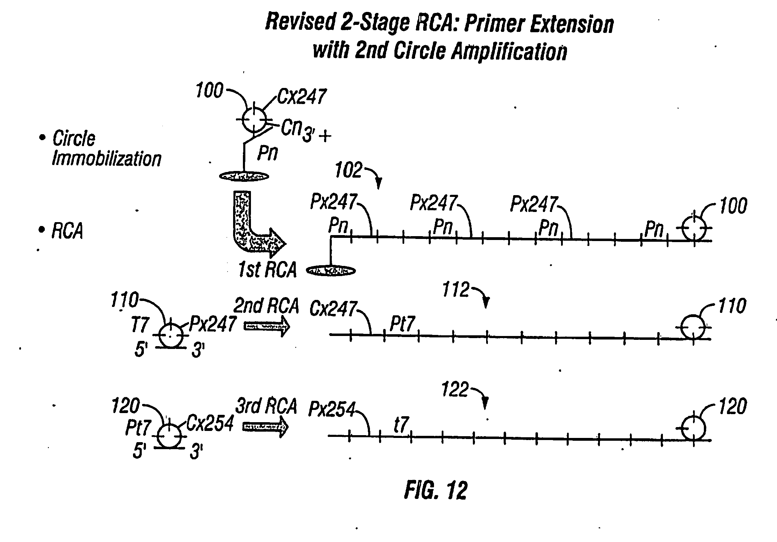

In the following example, a "two-stage" RCA procedure was employed in performing an electrochemical detection assay. This process is illustrated in FIG. 12. As shown, a synthetically prepared first circle 100 is captured by a capture probe and primer extension occurs. In FIG. 12, the circles and linear nucleic acid molecules are depicted as being segmented. Each segment is defined by the unique sequence of base pairs that it countains. When the RCA process occurs, the segments are repeated each time the circle completes a revolution. The first circle 100, for example, is shown as containing four segments, one of which is labeled "Cx247." The "Cx247" segment is complementary to the segment identified as "Px247" on the first amplicon 102 of the original immobilized strand. Another segment of the first circle is labeled "Cn." The "Cn" segment is complementary "Pn" segment on the amplicon 102.

In the next step, a secondary amplification occurs using an RCA initiation complex that contains a primer hybridized to a second circle 110. The second circle 110 shown in FIG. 12 contains three segments, two of which are designated "Px247" and "T7" respectively. This RCA initiation complex then carries out a second RCA process. Further, the product of the second RCA can hybridize to the product of the first RCA. As shown in FIG. 12, the first amplicon 102 contains segment Px247 and the second amplicon 112 contains sequence Cx247. Since Px247 and Cx247 are complementary, hybridization can occur between the two strands at those segments. The result of the hybridization is to effectively increase the amount of nucleic acid that is attached to the electrode and thereby augment the detectable electrical signal based on the presence of the nucleic acid.

Further, it is possible to use a "multistage" RCA procedure, wherein an additional RCA initiation complex (containing a primer hybridized to a third circular molecule) is utilized. As shown in FIG. 12, a third circle 120 is used in a second RCA initiation complex that can carry out a third RCA and produce a third amplicon 122. Here, the segment identified as "t7" is complementary to the segment identified as Pt7 on the second amplicon 112. As previously, the complementary regions can hybridize and the amount of nucleic acid attached to the electrode can be further increased. It is further contemplated that multistage RCA procedures can involve additional RCA initiation complexes and feature additional RCA procedures such as a fourth, fifth, sixth, and/or seventh RCA process or more. The RCA products of such procedures can be prepared such that they are able to hybridize to any other RCA product within the assay. It is advantageous that the various RCA products be designed so that each of them is able to hybridize to another strand such that each RCA product is either directly or indirectly attached to the original immobilized strand.

A two-stage RCA with detection by HRP was carried out as follows. Assay chips, each containing twelve electrodes were prepared by immobilizing capture probes on the electrode surfaces. The probes were selected because they are specific to a ligated PLP circle target to be interrogated. The chips were then soaked in 10 mM Hepes, pH 7.5/200 mM NaCl at 37 °C for 20 minutes. Square wave voltammetry (SWV) was run in 5 µM Ru(NH₃)₆³⁺ in 10 mM Tris/10 mM NaCl buffer. Target hybridization was performed by adding 20 µl of solution containing the target on 12-pad chips. The chips were incubated at 60 °C for 10 minutes and RT for 30 minutes. The chips were rinsed with 10 mM Tris/200 mM NaCl buffer. 20 µL per chip of RCA solution containing 50 mM Tris(pH 7.5) was added, along with 150 mM KCl, 10 mM Mg₂Cl, 4 mM DTT, 10 mM ammonium sulfate, 1.5 mM dNTPs, 400 nM of detector probe containing fluorescein tags, and 20 units of φ29. DNA polymerase. RCA then proceeded at room temperature for 5 minutes. 2 µl universal circle solution from ligation of 10 nM universal PLP and 15 nM RCA initiation complex was added to each chip and incubated at 37 °C for one hour. The chips were rinsed with PBS buffer. 50 µl of 1:200 diluted antifluorescein HRP conjugate in PBS/0.5% casein was added to each chip and incubated at RT for 30 minutes. The chips were rinsed with PBS/0.05% Tween 20. Steady state current was measured at -200 mV vs. Ag wire in K-Blue solution.

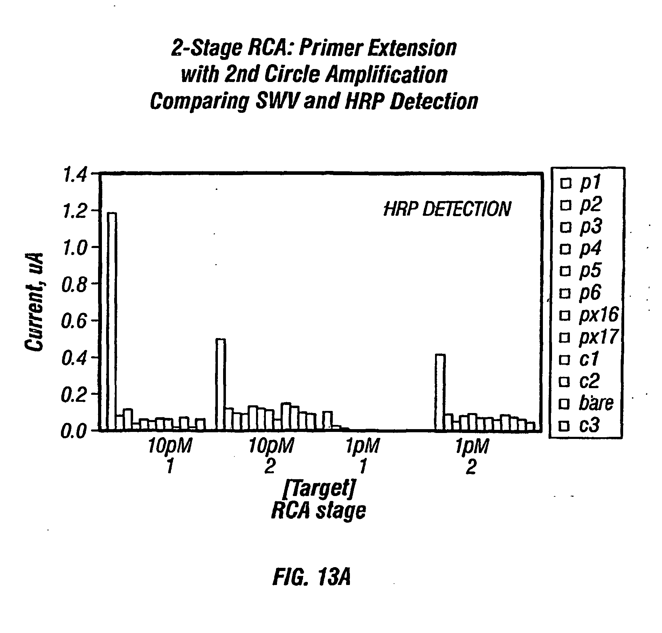

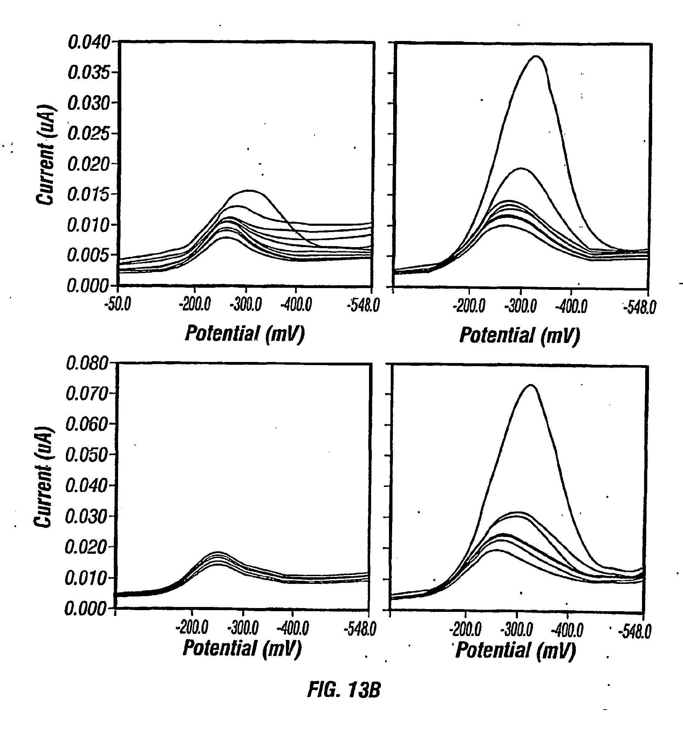

The results are shown in FIG. 13. As illustrated, observed current is higher following the second RCA stage than following the first RCA stage.

### Example 7: Two-Stage RCA with Detection Using Ruthenium Hexamine

A two-stage RCA with detection by square wave voltammetry (SWV) using Ru(NH₃)₆³⁺ was carried out as follows. Chips immobilized with capture probes specific to the ligated PLP circle as target were soaked in 10 mM Hepes, pH 7.5/200 mM NaCl at 37 °C for 20 minutes. Square wave voltammetry (SWV) was run in 5 µM Ru(NH₃)₆³⁺ in 10 mM Tris/10 mM NaCl buffer. Target hybridization was performed by adding 20 µl of solution containing the target on 12-pad chips. The chips were incubated at 60 °C for 10 minutes and RT for 30 minutes. The chips were rinsed with 10 mM Tris/200 mM NaCl buffer. 20 µL per chip of RCA solution containing 50 mM Tris(pH 7.5) was added, along with 150 mM KCl, 10 mM Mg₂Cl, 4 mM DTT, 10 mM ammonium sulfate, 1.5 mM dNTPs, and 20 units of φ29 DNA polymerase. RCA then proceeded at room temperature for 5 minutes. 2 µl universal circle solution from ligation of 10 nM universal PLP and 15 nM RCA initiation complex was added to each chip and incubated at 37 °C for 70 minutes. The chips were rinsed with PBS buffer. SWV was performed in 5 µM Ru(NH₃)₆³⁺ in 10 mM Tris/10 mM NaCl buffer.

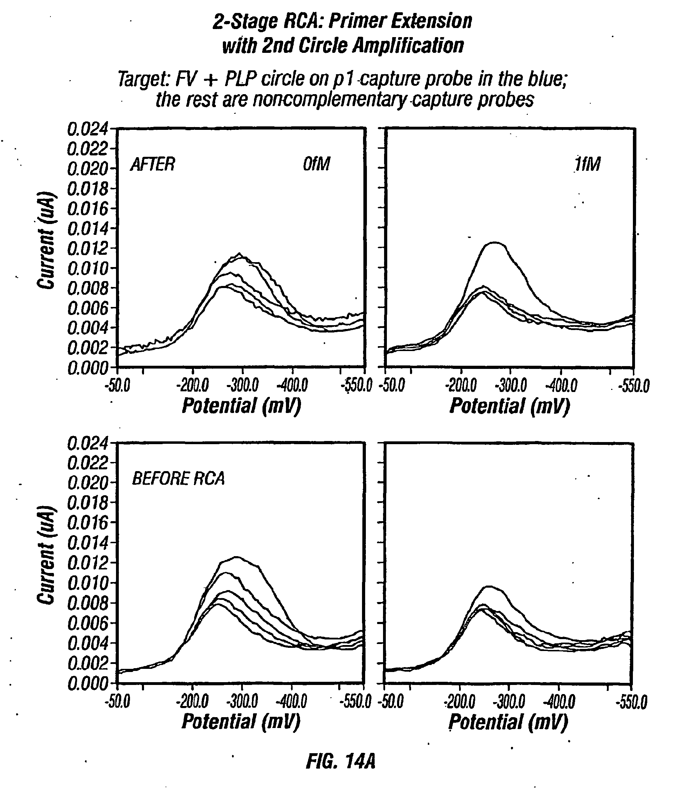

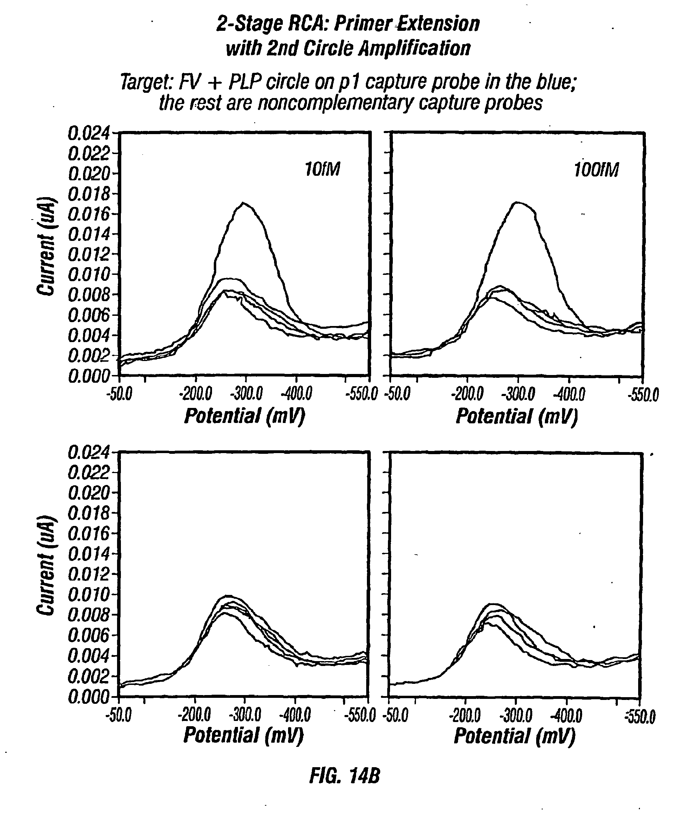

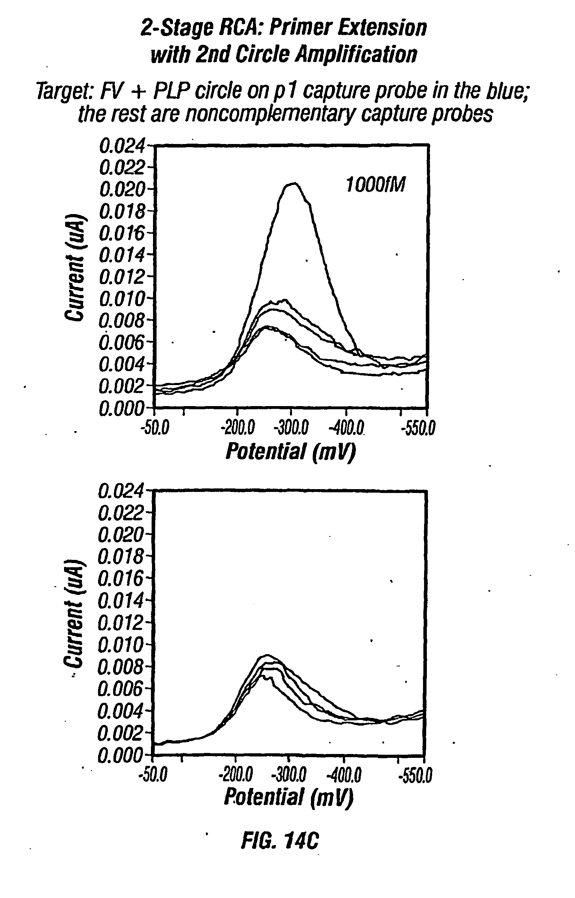

The results are shown in FIG. 14. As illustrated, observed current is higher after RCA than it is before RCA, and detectable current following RCA correlates with concentration.

By using the techniques described herein, ultra sensitive detection of DNA targets is made possible by bridging, elongation, catalytic detection, and various combinations thereof. On-chip RCA allows localized amplification and detection of targets. Accordingly, techniques for multiplexing DNA diagnostics as well as detection of bacteria or viruses can be performed which are superior to PCR or other types of amplification schemes.

In addition to electrochemical detection, some embodiments of the present invention can be used in other types of detection modes such as fluorescence, chemiluminescence, and colorimetry. Some embodiments can be used for microplate assays, microarray assays, membrane and filter assays, and electrochemical assays on various formats of electrode substrates.

Further, some embodiments of the invention can be used without target amplification, such as PCR and cell culture. For detection of bacteria, for example, one bacterial cell contains thousands to billions of copies of rRNA, each one with a length ranging from thousands to hundreds of thousands of nucleotides. With the advantages of multiple detector reagents per target molecule and enzyme catalyzed electrochemical detection, presently disclosed techniques may be used for rapid and direct detection of infectious diseases.

Further, some embodiments can be used to quantitate the level of a target nucleic acid in a sample. Such quantitation can be valuable in measuring the extent to which a pathogenic organism has proliferated in an individual. In particular, some embodiments are be useful in measuring the viral load in an infected individual.

## Claims

1. A method for determining whether a sample contains a target polynucleotide, comprising the steps of:
contacting said sample with a detection zone comprising a capture probe polynucleotide complementary to at least a portion of a sequence in said target polynucleotide under conditions which permit said target polynucleotide to hybridize to said capture probe polynucleotide, wherein said detection zone comprises an electrode;
extending said target polynucleotide;
hybridizing a first bridge nucleic acid to the extended target polynucleotide hybridized to said capture probe polynucleotide, wherein said first bridge nucleic acid comprises a nucleotide sequence complementary to a nucleotide sequence in said target polynucleotide and a nucleotide sequence complementary to a first circular nucleic acid;
extending said first bridge nucleic acid such that said extended first bridge nucleic acid is non-contiguous with said probe polynucleotide; and
determining whether a signal indicative of the presence of said target polynucleotide in said sample is generated, wherein said signal is generated by a catalytic detection reagent which can produce a plurality of signals without being exhausted.

2. The method of Claim 1 wherein said target polynucleotide comprises a nucleic acid sequence from a biological sample.

3. The method of Claim 1 wherein said target polynucleotide comprises a nucleic acid tag sequence.

4. The method of Claim 1, wherein the extending comprises performing rolling circle amplification.

5. The method of Claim 1, further comprising hybridizing a second bridge nucleic acid to said extended first bridge nucleic acid, wherein said second bridge comprises a nucleotide sequence complementary to a nucleotide sequence in said extended first bridge nucleic acid and a nucleotide sequence complementary to a second circular nucleic acid.

6. The method of Claim 1 wherein the catalytic detection reagent comprises a peroxidase.

7. The method of Claim 6 wherein the peroxidase is horseradish peroxidase.

## Patentansprüche

1. Verfahren zum Bestimmen, ob eine Probe ein Ziel-Polynucleotid enthält, das die folgenden Schritte beinhaltet:
Inkontaktbringen der genannten Probe mit einer Detektionszone, die ein Fangsonden-Polynucleotid enthält, das zu wenigstens einem Teil einer Sequenz in dem genannten Ziel-Polynucleotid komplementär ist, unter Bedingungen, bei denen das genannte Ziel-Polynucleotid mit dem genannten Fangsonden-Polynucleotid hybridisieren kann, wobei die genannte Detektionszone eine Elektrode enthält;
Verlängern des genannten Ziel-Polynucleotids;
Hybridisieren einer ersten Brücken-Nucleinsäure mit dem verlängerten Ziel-Polynucleotid, das mit dem genannten Fangsonden-Polynucleotid hybridisiert ist, wobei die genannte erste Brücken-Nucleinsäure eine Nucleotidsequenz, die zu einer Nucleotidsequenz in dem genannten Ziel-Polynucleotid komplementär ist, und eine Nucleotidsequenz enthält, die zu einer ersten zirkulären Nucleinsäure komplementär ist;
Verlängern der genannten ersten Brücken-Nucleinsäure, so dass die genannte verlängerte erste Brücken-Nucleinsäure nicht an das genannte Sonden-Polynucleotid angrenzt; und
Ermitteln, ob ein Signal, das auf die Anwesenheit des genannten Ziel-Polynucleotids in der genannten Probe hindeutet, erzeugt wird, wobei das genannte Signal durch ein katalytisches Detektionsreagenz erzeugt wird, das mehrere Signale produzieren kann, ohne zu erschöpfen.

2. Verfahren nach Anspruch 1, wobei das genannte Ziel-Polynucleotid eine Nucleinsäuresequenz von einer biologischen Probe enthält.

3. Verfahren nach Anspruch 1, wobei das genannte Ziel-Polynucleotid eine Nucleinsäure-Tag-Sequenz enthält.

4. Verfahren nach Anspruch 1, wobei das Verlängern das Durchführen einer Rolling-Circle-Amplifikation beinhaltet.

5. Verfahren nach Anspruch 1, das ferner das Hybridisieren einer zweiten Brücken-Nucleinsäure mit der genannten verlängerten ersten Brücken-Nucleinsäure beinhaltet, wobei die genannte zweite Brücke eine Nucleotidsequenz, die zu einer Nucleotidsequenz in der genannten verlängerten ersten Brücken-Nucleinsäure komplementär ist, und eine Nucleotidsequenz enthält, die zu einer zweiten zirkulären Nucleinsäure komplementär ist.

6. Verfahren nach Anspruch 1, wobei das katalytische Detektionsreagenz eine Peroxidase beinhaltet.

7. Verfahren nach Anspruch 6, wobei die Peroxidase Meerrettichperoxidase ist.

## Revendications

1. Procédé pour déterminer si un échantillon contient un polynucléotide cible, comprenant les étapes consistant à :
mettre en contact ledit échantillon avec une zone de détection comprenant un polynucléotide sonde de capture complémentaire à au moins une partie d'une séquence dans ledit polynucléotide cible dans des conditions qui permettent audit polynucléotide cible de s'hybrider audit polynucléotide sonde de capture, où ladite zone de détection comprend une électrode ;
prolonger ledit polynucléotide cible ;
hybrider un premier acide nucléique de pontage au polynucléotide cible prolongé hybridé audit polynucléotide sonde de capture, où ledit premier acide nucléique de pontage comprend une séquence nucléotidique complémentaire à une séquence nucléotidique dans ledit polynucléotide cible et une séquence nucléotidique complémentaire à un premier acide nucléique circulaire ;
prolonger ledit premier acide nucléique de pontage de telle sorte que ledit premier acide nucléique de pontage prolongé soit non contigu audit polynucléotide sonde ; et
déterminer si un signal indicatif de la présence dudit polynucléotide cible dans ledit échantillon est généré, où ledit signal est généré par un réactif de détection catalytique qui peut produire une pluralité de signaux sans être épuisé.

2. Procédé de la Revendication 1 où ledit polynucléotide cible comprend une séquence d'acide nucléique provenant d'un échantillon biologique.

3. Procédé de la Revendication 1 où ledit polynucléotide cible comprend une séquence marqueur d'acide nucléique.

4. Procédé de la Revendication 1, où le prolongement comprend la réalisation d'une amplification par cercle roulant.

5. Procédé de la Revendication 1, comprenant en outre l'hybridation d'un second acide nucléique de pontage audit premier acide nucléique de pontage prolongé, où ledit second pontage comprend une séquence nucléotidique complémentaire à une séquence nucléotidique dans ledit premier acide nucléique de pontage prolongé et une séquence nucléotidique complémentaire à un second acide nucléique circulaire.

6. Procédé de la Revendication 1 où le réactif de détection catalytique comprend une peroxydase.

7. Procédé de la Revendication 6 où la peroxydase est de la peroxydase de raifort.


### SEQUENCE LISTING

<110> Crothars, Donald M. Holmlin, R. Erik zhang, Honghua Shi, Chunnian
<120> NUCLEIC ACID DETECTION METHOD HAVING
   INCREASED SENSITIVITY
<130> GENOM.034VPC
<150> 60/518,816
   <151> 2003-11-10
<160> 26
<170> FastSEQ for Windows Version 4.0
<210> 1
   <211> 42
   <212> DNA
   <213> Artificial Sequence
<220>
   <223> Synthetic oligonucleotide sequence
<400> 1
   gggaggagct gaccagtgaa gaaagtgtct ttgaagtctt tg 42
<210> 2
   <211> 72
   <212> DNA
   <213> Artificial Sequence
<220>
   <223> Synthetic oligonucleotide sequence
<221> misc_feature
   <222> (23)...(42)
   <223> T7
<221> misc_feature
   <222> (43)...(57)
   <223> CX16
<400> 2
   cttcactggt cagctcctcc caccctatag tgagtcgtat tatctacctt gagacacctt 60
   caaagacact tt 72
<210> 3
   <211> 52
   <212> DNA
   <213> Artificial sequence
<220>
   <223> Synthetic oligonucleotide sequence
<400> 3
   tctgtaagag cagatccctg gacaggcaag gaatacaggt attttgtcct tg 52
<210> 4
   <211> 58
   <212> DNA
   <213> Artificial Sequence
<220>
   <223> Synthetic oligonucleotide sequence
<221> misc_feature
   <222> (27) ... (42)
   <223> C2
<400> 4
   gactgtccag ggatctgctc ttagctcgta gttatccgta ccaatacctg tattcctt 58
<210> 5
   <211> 30
   <212> DNA
   <213> Artificial Sequence
<220>
   <223> Synthetic oligonucleotide sequence
<400> 5
   attccttgcc tgtccgtgtc tcaaggtaga 30
<210> 6
   <211> 80
   <212> DNA
   <213> Artificial Sequence
<220>
   <223> Synthetic oligonucleotide sequence
<400>
   attccttgcc tgtccaaaaa aaaaaaaaaa aaaaaaaaaa aaaaaaaaaa aaaaaaaaaa 60
   aaaaagtgtc tcaaggtaga 80
<210> 7
   <211> 70
   <212> DNA
   <213> Artificial Sequence
<220>
   <223> Synthetic oligonucleotide sequence
<400> 7
   ctgttgttga tactacacgc agtacaaata tgtcattatg tgctgccata tctacttcag 60
   aaactacata 70
<210> 8
   <211> 73
   <212> DNA
   <213> Artificial Sequence
<220>
   <223> Synthetic oligonucleotide sequence
<400> 8
   atgtcattat gtgctgccat atctacttca gaaactacat ataaaaatac taactttaag 60
   gagtacctac gac 73
<210> 9
   <211> 26
   <212> DNA
   <213> Artificial Sequence
<220>
   <223> synthetic oligonucleotide sequence
<221> misc_feature
   <222> 26
   <223> 3 biotinylation
<400> 9
   gtagatatgg cagcacaaaa aaaaaa 26
<210> 10
   <211> 46
   <212> DNA
   <213> Artificial Sequence
<220>
   <223> Synthetic oligonucleotide sequence
<221> misc_feature
   <222> 46
   <223> 3' biotinylation
<400> 10
   gtagatatgg cagcacaaaa aaaaaaaaaa aaaaaaaaaa aaaaaa 46
<210> 11
   <211> 39
   <212> DNA
   <213> Artificial Sequence
<220>
   <223> Synthetic oligonucleotide sequence
<400> 11
   actgcgtgta gtatcattat attggtacgg ataactacg 39
<210> 12
   <211> 41
   <212> DNA
   <213> Artificial Sequence
<220>
   <223> Synthetic oligonucleotide sequence
<400> 12
   cgtaggtact ccttaaagtt atattggtac ggataactac g 41
<210> 13
   <211> 37
   <212> DNA
   <213> Artificial Sequence
<220>
   <223> Synthetic oligonucleotide sequence
<400> 13
   cctgtattcc ttgcctgtcc agggatctgc tcttacc 37
<210> 14
   <211> 38
   <212> DNA
   <213> Artificial Sequence
<220>
   <223> Synthetic oligonucleotide sequence
<400> 14
   ctatagtgag tcgtattacg tagttatccg taccaata 38
<210> 15
   <211> 39
   <212> DNA
   <213> Artificial Sequence
<220>
   <223> Synthetic oligonucleotide sequence
<400> 15
   actgcgtgta gtatcattat attggtacgg ataactacg 39
<210> 16
   <211> 70
   <212> DNA
   <213> Artificial Sequence
<220>
   <223> Synthetic oligonucleotide sequence
<400> 16
   ctgttgttga tactacacgc agtacaaata tgtcattatg tgctgccata tctacttcag 60
   aaactacata 70
<210> 17
   <211> 26
   <212> DNA
   <213> Artificial Sequence
<220>
   <223 Synthetic oligonucleotide sequence
<400> 17
   gtagatatgg cagcacaaaa aaaaaa 26
<210> 18
   <211> 41
   <212> DNA
   <213> Artificial Sequence
<220>
   <223> Synthetic oligonucleotide sequence
<400> 18
   cgtaggtact ccttaaagtt atartggtac ggataactac g 41
<210> 19
   <211> 73
   <212> DNA
   <213> Artificial Sequence
<220>
   <223> Synthetic oligonucleotide sequence
<400> 19
   atgtcattat gtgctgccat atctacttca gaaactacat ataaaaatac taactttaag 60
   gagtacctac gac 73
<210> 20
   <211> 26
   <212> DNA
   <213> Artificial Sequence
<220>
   <223> synthetic oligonucleotide sequence
<400> 20
   gtagatatgg cagcacaaaa aaaaaa 26
<210> 21
   <211> 26
   <212> DNA
   <213> Artificial Sequence
<220>
   <223> Synthetic oligonucleotide sequence
<221> misc_feature
   <222> 26
   <223> 3' biotinylation
<400> 21
   gtagtateta ccacagaaaa aaaaaa 26
<210> 22
   <211> 39
   <212> DNA
   <213> Artificial Sequence
<220>
   <223> synthetic oligonucleotide sequence
<221> misc_feature
   <222> 1
   <223> 5 biotinylation
<400> 22
   aaaaaaaaaa aaaaaaaaaa aaaaagtaca tttagacge 39
<210> 23
   <211> 75
   <212> DNA
   <213> Artificial Sequence
<220>
   <223> synthetic oligonucleotide sequence
<400> 23
   taatcaatta tttcttactg tggtagatac taccatcggg aggaatatga tttacagttt 60
   atttttcgtt gtgta 75
<210> 24
   <211> 43
   <212> DNA
   <213> Artificial Sequence
<220>
   <223> synthetic oligonucleotide sequence
<400> 24
   aaactgtaaa tcatattcct ccctattggt acggataact acg 43
<210> 25
   <211> 75
   <212>
   <213> Artificial Sequence
<220>
   <223> Synthetic oligonucleotide sequence
<400> 25
   gcctgtccag ggatctgctc ttaccctata gtgagtcgta ttacgtagtt atccgtacca 60
   atacctgtat tcctt 75
<210> 26
   <211> 65
   <212> DNA
   <213> Artificial Sequence
<220>
   <223> Synthetic oligonucleotide sequence
<400> 26
   aaaaaaaaaa aaaaaaaaaa aaaaaaaaaa aaaaaaaaaa aaaaaaaaaa gtacggataa 60
   ctacg 65